# EUROPEAN PATENT SPECIFICATION

(11) **EP 2 452 189 B1**
(45) Date of publication and mention of the grant of the patent: **16.10.2013**
(21) Application number: 10730456.0
(22) Date of filing: 05.07.2010
(51) Int. Cl.: G01N 33/50, A61K 48/00

(54) **PROCESS FOR THE IDENTIFICATION OF COMPOUNDS FOR TREATING CANCER**
VERFAHREN FÜR DIE IDENTIFIZIERUNG VON VERBINDUNGEN ZUR BEHANDLUNG VON KREBS
PROCÉDÉ D'IDENTIFICATION DE COMPOSÉS DESTINÉS À TRAITER LE CANCER

(30) Priority: 04.07.2009 ES 200930417
(43) Date of publication of application: 16.05.2012
(73) Proprietor: Fundación Centro Nacional De Investigaciones Oncológicas Carlos III, 28029 Madrid (ES)
(72) Inventor: SOENGAS GONZÁLEZ, María Soledad, E-28029 Madrid (ES); TORMO CARULLA, Damià, E-28029 Madrid (ES)
(74) Representative: Illescas, Manuel
(86) International application number: PCT/EP2010/059593
(87) International publication number: WO 2011/003883

(56) References cited:
- WO-A2-2004/045491
- WO-A2-2006/001956
- US-A1- 2005 244 505
- US-A1- 2007 259 372
- KANG D-C ET AL: "mda-5: An interferon-inducible putative RNA helicase with double-stranded RNA-dependent ATPase activity and melanoma growth-suppressive properties" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.022637199, vol. 99, no. 2, 22 January 2002 (2002-01-22), pages 637-642, XP008122805 ISSN: 0027-8424
- SUN YAPING ET AL: "Involvement of Noxa in cellular apoptotic responses to interferon, double-stranded RNA, and virus infection" JOURNAL OF BIOLOGICAL CHEMISTRY, vol. 280, no. 16, April 2005 (2005-04), pages 15561-15568, XP002597294 ISSN: 0021-9258
- MOORE HANNAH E ET AL: "Aminopeptidase inhibition as a targeted treatment strategy in myeloma" MOLECULAR CANCER THERAPEUTICS, vol. 8, no. 4, April 2009 (2009-04), pages 762-770, XP002597295 ISSN: 1535-7163
- TORMO DAMIA ET AL: "Targeted Activation of Innate Immunity for Therapeutic Induction of Autophagy and Apoptosis in Melanoma Cells" CANCER CELL, vol. 16, no. 2, August 2009 (2009-08), pages 103-114, XP002597296 ISSN: 1535-6108
- ALONSO-CURBELO DIRENA ET AL: "Self-killing of melanoma cells by cytosolic delivery of dsRNA Wiring innate immunity for a coordinated mobilization of endosomes, autophagosomes and the apoptotic machinery in tumor cells" AUTOPHAGY, vol. 6, no. 1, January 2010 (2010-01), pages 148-150, XP9137677 ISSN: 1554-8627

### SEQUENCE LISTING

<110> Fundación Centro Nacional de Investigaciones Oncológicas Carlos III.
<120> Process for the identification of compounds for treating cancer.
<130> PCT-03837
<160> 3
<170> PatentIn version 3.3
<210> 1
   <211> 21
   <212> DNA
   <213> Homo sapiens
<220>
   <221> misc feature
   <222> (1)..(21)
   <223> Target sequence of shRNA used to down regulate MDA-5.
<400> 1
   cgcaaggagt tccaaccatt t 21
<210> 2
   <211> 19
   <212> DNA
   <213> Artificial
<220>
   <223> RT-PCR forward primer to detect MDA-5 expression.
<400> 2
   ggcaccatgg gaagtgatt 19
<210> 3
   <211> 20
   <212> DNA
   <213> Artificial
<220>
   <223> RT-PCR reverse primer to detect MDA-5 expression.
<400> 3
   atttggtaag gcctgagctg 20

## Description

### TECHNICAL FIELD

The present invention relates to the oncology field and it is mainly focused on indentifying compounds that can be used for treating different types of cancer, for example: melanoma, pancreas, colon, bladder, glioma, breast, prostate, lung and ovarian carcinoma.

Moreover, the present invention also covers the compounds identified by such a process, for example the compound BO-110 (see below), which is able to promote a clear tumor cell demise in all above indicated types of cancer.

### STATE OF THE ART

Melanoma remains a prototype of solid cancers with increasing incidence and extremely poor prognosis at advanced stages (Jemal et al., 2008). Considerable effort has been devoted to the identification of molecular determinants underlying melanoma chemo- and immunoresistance. The only agents approved by the US Food and Drug Administration (FDA) for the treatment of metastatic melanoma are the alkylating agent dacarbazine (DTIC) and the immunomodulator IL-2 (Tawbi and Kirkwood, 2007). Yet, durable and complete responses in metastatic melanoma rarely benefit more than 5% of patients, and secondary toxicities can be severe. Consequently, current average survival of patients with metastatic melanoma is 6 to 10 months, and therefore, the development of improved therapies is a priority in this disease (Jemal et al. 2007).

Initially, the viral dsRNA synthetic analogue called pIC (polyinosine-polycytydylic acid), a compound that has been used for more than four decades to stimulate the immune system independently of interferon (IFN) (Field et al. 1967), was thought to be a promising therapeutic agent against melanoma. Unfortunately, the clinical studies with naked pIC revealed its low stability, low induction of IFN and absence of antitumoral effect for melanoma (Robinson et al., 1976). Thus, as a single agent pIC was considered a poor agent for melanoma.

High throughput histogenetic analyses and systematic functional studies have significantly advanced our understanding of melanoma initiation and progression and the complex mechanisms associated with treatment failure (Fecher et al., 2007; Gray-Schopfer et al., 2007). Consistent defects and alterations in BRAF/MAPK; PI3K/AKT, NF-κB or NOTCH signaling cascades are being identified, providing an exciting platform for rational drug design (Gray-Schopfer et al., 2007). However, targeted therapy has not yet been proven effective in melanoma trials (Flaherty, 2006). Death programs controlled by mitochondria and/or by the endoplasmic reticulum are also under evaluation, although are invariably ineffective in vivo (Hersey and Zhang, 2008). Consequently, current anticancer drugs either do not reach their target(s) in a productive manner or have to be administered at dosing schedules that result in unbearable toxicities to normal cellular compartments (Tawbi and Kirkwood, 2007). Importantly, compensatory mechanisms can be activated during treatment, selecting for cell populations with an even higher chemoresistance (Lev et al., 2004; Shatton et al., 2008; Wolter et al., 2007)

In fact, melanoma is considered to have a strong capacity to evade apoptosis through different pathways, which confers melanoma the capacity to progress, form metastasis and survive treatment with different therapies (reviewed by Ivanov et al., 2003)

In contrast to standard chemotherapy, which aims to kill tumor cells primarily from "within" (i.e. by activating intrinsic programs of cell death), immunotherapy has traditionally involved an indirect cascade of cell-cell interactions. In melanoma, most efforts have focused on boosting the levels or functional efficacy of two compartments: antigen presenting cells and cytotoxic T cells (Wilcox and Markovic, 2007). Vaccines as well as antibodies directed against inhibitory immunomodulators (e.g. CTL4) are also being tested, although with frustrating results in phase IV clinical trials (Kirkwood et al., 2008). More recently, stimulation of the innate immune system via activation of Toll Like Receptors (TLR)-3, -4, -7 and -9, is pursued to support cytotoxic destruction of melanoma cells by natural killer (NK), dendritic cells (DC) and T cells (Kirkwood et al., 2008, and Tormo et al. 2007). However, multiple studies, including our own, have demonstrated an inherent ability of cells to bypass immunological therapies by downregulating (editing) immunoreactive surface markers. Melanomas can also exert suppressive effects on the host (e.g. inhibition of the maturation of antigen-presenting cells or blockade of full T-cell activation) (Tormo et al., 2006; Ilkovitch and Lopez, 2008; Verma et al., 2008). Thus, melanomas present an inherited capacity to elude the antitumoral activity of immunommodulators.

In the field of immunotherapy, one of the molecules which its increase has been studied as a potential positive factor for the therapy of melanoma is MDA-5 (Melanoma Differentiation Associated Gen 5), a product initially described as a gene associated with melanoma differentiation (Kang et al., 2002). MDA-5 is a helicase that recognizes and is activated by long double stranded RNA (dsRNA) (Yoneyama et al., 2005). Other RNA helicases are RIG-1 (retinoic acid inducible protein 1, also called Dsx58), which recognizes naked 3 phosphates of short dsRNA, and LGP2 (also called Dhx58), which is a negative regulator in dsRNA sensing.

As long dsRNAs can be generated by and during viral infections, MDA-5 acts as a first line of innate immunity against viral pathogens (Akira et al., 2006). Moreover, MDA-5 has caspase activation recruitment domains (CARD). Together, the helicase and the CARD domains activate NF-κB and other transcription factors implicated in cytokine regulation (Kawai et al., 2005). Thus, the best known function of MDA-5 is immune stimulation.

From a therapeutic prospective, it is known that both IFN-β and dsRNA induce the transcription of the Mda-5 gen. Therefore dsRNA has been proposed to have a role in the increase of the Mda-5 expression in the IFN-induced growth inhibition. In addition, it has been shown (Kang et al., 2002) that the induction of endogenous MDA-5 by IFN-β is cytostatic (in other words, stops the cell cycle). Thus, to activate tumor cell death, MDA-5 had to be overexpressed ectopically at high levels (Kovacsovics et al., 2002). Furthermore, this pro-apoptotic activity of the ectopic expression of MDA-5 is not efficient in tumor cells with hyperactive RAS/MEK/ERK pathway (Lin et al., 2006), as is the case of melanomas (Chin et al., 2006). Thus, a pending question in the field was how to activate the endogenous MDA-5 with chemotherapeutic agents in a manner exclusive to the tumor compartment (without inducing secondary toxicities in normal cells).

The U.S. patent application US 2007/0259372 proposes the identification of agonists or antagonists of IFN-β, IFN-α or IFN-γ by compounds capable of enhancing the expression of MDA-5. This patent also suggests that the inducers of Mda-5 gene expression (by means of its promoter) can be considered as candidate compounds for induced terminal differentiation of tumor cells. It also suggests a possible role of MDA-5 in the generation of apoptotic signals through its CARD domain. However, so far, it was not known which were the targets of MDA-5 that can trigger apoptosis, and how to activate it in a traceable and selective way for tumor cells. Moreover, as melanoma cells have an active RAS/MEK/ERK active pathway (which inhibits MDA-5), as well as a marked ability to circumvent apoptotic cell death, it was not obvious that apoptotic signals mediated by MDA-5 would be a valid mechanism for therapy against melanoma. Therefore, from the previous information about MDA-5 regulation and function, this protein did not appear as a strong target for procedures to identify candidates for therapeutic agents against melanoma.

Autophagy is emerging as an alternative strategy to engage the endogenous death machinery of cancer cells.

This process involves an intricate cascade of events that ultimately leads to the sequestration of cytosolic components for subsequent degradation by the lysosome (Xie and Klionsky, 2007). Depending on the mechanism of engulfment and the nature of the cargo delivered to the autolysosomes, multiple mechanisms of autophagy have been described. In the context of anticancer treatment, macroautophagy, or bulk degradation of cellular organelles and protein aggregates, is raising interest for its potential to compromise cell viability by dysfunction or excessive depletion of key organelles (e.g. endoplasmic reticulum or mitochondria) (Hoyer-Hansen, 2008).

However, it is unknown how autophagy is regulated, and its therapeutic potential is not clear and simple (Hippert et al., 2006). On the one hand, macroautophagy (which we will refer simply as "autophagy" hereafter) has demonstrated significant potential to protect cells against a wide variety of aggressive intracellular and extracellular signals, including anticancer drugs. By this activity, autophagy can promote tumor development (Mizushima et al., 2008; Kroemer and Levine, 2008).

Paradoxically, autophagy has also been associated with cell death (Kromer et al. 2009). Thus, excessive or persistent autophagy can promote cell killing by depletion of key organelles (i.e. endoplasmic reticulum or mitochondria), rewiring of survival signals, deregulation of lysosomal enzymes, and/or activation of caspase-dependent apoptotic programs (Xie and Klionsky, 2007).

Consequently, it was unclear whether autophagy would exacerbate melanoma chemo- and immune-resistance, instead of improving treatment response. Furthermore, none of the more than 20 autophagy genes described up to date in mammalian cells, has been characterized in detail in melanoma. Therefore, whether autophagy is regulated in a differential manner in melanoma and normal cells to provide a window for therapeutic intervention is unknown. A similar situation applies to aggressive cancers such as those affecting pancreas, bladder, prostate and brain.

WO2006/001956 discloses methods for evaluating the ability of a test molecule to induce NOXA in a cancer cell comprising contacting the cell with the test molecule and determining whether the cell exhibits and increase in NOXA, relative to a control.

WO2004/045491 discloses a compound comprising a polycation (PEI) and dsRNA (pIC) with a range of 10-3000 ribonucleotides, wherein the dsRNA (pIC) actually used for performing all the examples is 100-300 ribonuclotides in length (it is obtained from Pharmacia-Amersham).

In this situation, it remains the identification of therapeutic agents for the treatment of cancer, alternative to those already authorized and, specially, that are valid for the treatment of immunocompromised patients. It also remains necessary to identify possible new therapeutic targets for the development of procedures for identifying candidate therapeutic agents for the treatment of cancer among the compounds capable of acting on these targets.

Thus, the current invention presents a solution for both problems.

### DESCRIPTION OF THE INVENTION

As cited above, the present invention is firstly focused on the identification of therapeutic targets, markers, or parameters, which set the basis for the development of a process useful for the identification of compounds (among those capable of acting on these therapeutic targets, markers or parameters) able to treat the cancer.

One of the markers identified in the present invention, useful for the identification of compounds able to treat the cancer, is the level of activation of the family helicase MDA-5. This parameter can be determined by checking the existence of proteolytic cleavage of the protein that results in the separation of the helicase and caspase domains: the candidate compound, to be a therapeutic agent for the treatment of cancer should result in the proteolytic cleavage, which is an indication that it can lead to the activation of autophagy and apoptosis mechanisms that would result in the death of cancer cells. A possible methodology for this test are immunoblotting (Western blotting) of cell culture protein extracts and testing the signal bands corresponding to the whole protein and fragments corresponding to the helicase domains and caspase domains. Specifically, as shown in Example 3, the appearance of a band of 30 kD is indicative of the existence of proteolytic cleavage. Alternatively, it could also determine the activation of other helicases family of MDA-5, such as RIG-I or LGP2.

Another marker identified in the present invention, also useful for the identification of compounds able to treat the cancer, is the level of NOXA expression. The rationale behind is an increase in the levels of expression of the corresponding genes when the mechanisms of autophagy and apoptosis are activated. The determination of the expression levels of these proteins can be performed, for example, determining the concentration of the corresponding messenger RNA (this can be carried out, for example, by Northern Blot or RT-PCR), or the concentration of the protein itself in a protein extract of the corresponding cell culture (for example, by a transfer like Western Blot). Moreover, NOXA can be detected in situ (in tissue specimens), by immunohisto chemistry.

In a preferred embodiment of the present invention, MDA-5 and NOXA are both examined, as corroboration that the mechanisms of autophagy and apoptosis are activated, as MDA-5 is considered a point of link between them.

Additionally, the invention may include a step for determining the induction of autophagy by the candidate compound to be used against cancer. The induction of autophagy can be determined by several techniques, which comprise:
- Monitoring the posttranslational modifications of the protein expressed by the autophagy gene 8 (which is referred to ATG8 or LC3). LC3 protein is processed and lipidated when inserted into autophagosomes, which are membranous structures where cellular components are kidnapped during autophagy. Due to the conformation and electrophoretic mobility of this protein are changed by lipidation, one of the possible techniques to verify the induction of autophagy is the use of immunoblot techniques with antibodies directed against this protein. This technique allows verifying that the band corresponding to the protein, after performing the electrophoresis, is different in control samples as compared with the samples in which it is supposed that the compound has induced autophagy. Alternatively, if the antibody specifically recognizes the autophagosomes form, the union of the antibody would confirm the induction of autophagy in the sample treated with the candidate compound.
- Monitoring of changes in intracellular distribution of LC3 protein, because another hallmark of autophagy is the relocation of LC3 from the cytosol to the newly formed autophagosomes (Xie and Klionsky, 2007). Thus the formation of protein foci can be considered indicative of the formation of autophagosomes, especially in early stages. This can be detected by monitoring the endogenous LC3 by immunofluorescence or immunohistochemistry on fixed cells or fixed tissues. Alternatively, autophagy can be visualized in living cells by determining the cellular localization fluorescent derivative of LC3. It is common to use as fluorescent protein GFP (green fluorescent protein) or RFP (red fluorescent protein), which allow tracking of autophagy by fluorescence microscopy: changes in cell fluorescence distribution from a diffuse pattern to a focal staining are indicative of the induction of autophagy. In the present invention, this methodology involves the use of cells that had been either transfected with a vector that allowed transient expression of the fusion protein (such as a plasmid or recombinant virus), or cells where the DNA segment, capable of expressing the fusion protein formed by the reporter protein and LC3, were integrated into the genome in a stable manner. An example of this strategy is showed in the Example 1 below, where cells were previously transfected with a recombinant retrovirus. This resulted in the insertion, into the cellular genome, of the DNA fragment containing the coding parts of the protein GFP and LC3 together, in a way that it would lead to a fusion protein into the cellular genome DNA. Thus, the tests of intracellular distribution changes with the stable transfected cells could be carried out.
- Use of transmission electron microscopy to detect the entry of a candidate compound into the cell. This situation directs the autophagosome formation in the more advanced stages of the process of autophagy. The visualization of dense structures accumulation (membrane-bound) is considered an indicative feature of autophagosome formation. The process of autophagy can be confirmed in later stages of the process (i.e. 24 or 30 hours after the treatment with the compound to be tested), at which the electron microscope should show the formation of large phagocytic vacuoles, indicative of cellular collapse.

Bearing in mind the above discussion, this description refers to a process, for the identification of compounds to be used for treating cancer, comprising the steps of:
a) Contacting the candidate compound with a cancer cell culture, or cancer cell line derived from cancer cells;
b) Determining at least one of the following parameters:
   i. Level of activation of a family helicase MDA-5;
   ii. The level of NOXA expression;
   iii. Or a combination thereof;
c) Comparing the data obtained in step b) with those observed in control of the same cells treated similarly, but in the absence of the candidate compound;
d) Selecting the compounds which have given rise to a significant increase in the parameter or parameters determined in step b) in comparison with the control.

It should be noted that the difference between the data obtained from cell culture treated with the candidate compound and the untreated control will be considered statistically significant when the analysis results in values of p <0.05.

In a preferred embodiment, the process of the invention also determines whether the candidate compound induces autophagy in cancer cells, in a cell line derived from cancer cells, or in a cancer model mouse. As explained above, the determination of the autophagy induction may be performed by checking the level of expression, the presence of posttranslational modifications or intracellular localization of an autophagy protein. More specifically the induction of autophagy is determined by a technique selected from: change of electrophoretic mobility of the protein LC3 or detection of foci formation of protein LC3. Alternatively the induction of autophagy is determined by checking the presence of autophagosomes by microscopic observation thereof, for example using transmission electron microscopy.

In a further preferred embodiment, the above described process of the invention comprises three steps: determination of the activation level of MDA-5, the level of expression of NOXA and the induction of autophagy.

The process of the invention may be used for the identification of compounds to be used as therapeutic agents for treating several types of cancer, for example: melanoma, pancreas, colon, bladder, breast, prostate, lung and ovarian carcinoma.

Therefore, if the process herein described aims to identify compounds to be used as therapeutic agents for treating melanoma, it will comprise the following steps:
a) Contacting the candidate compound with a melanoma cell culture, or a cell line derived from melanoma cells;
b) Determining at least one of the following parameters:
   i. Level of activation of a family helicase MDA-5;
   ii. The level of NOXA expression;
   iii. Or a combination thereof;
c) Comparing the data obtained in step b) with those observed in control of the same cells treated similarly, but in the absence of the candidate compound;
d) Selecting the compounds which have given rise to a significant increase in the parameter or parameters determined in step b) in comparison with the control.

As for valid cell lines, it can be used from any melanoma cell line, preferably from a human origin. Examples of valid cell lines, which are used in the examples of the invention, are human cell lines SK-Mel-19, SK-Mel-28, SK-Mel-103 and SK-Mel-147, and the murine B16 cells. Normal cell controls, melanocytes or other skin cells, as well as cells of the immune system, which usually represent sites of secondary toxicity in cancer treatment.

Alternatively, the process herein described may be focused on the identification of compounds to be used as therapeutic agents for treating at least one of the following types of cancer: pancreas, colon, bladder, breast, prostate, lung and ovarian carcinoma.

In this case the process would comprise the following steps:
a) Contacting the candidate compound with a cancer cell culture from at least one of the above cited types of cancer, or a cell line derived from at least one of the above cited types of cancer;
b) Determining at least one of the following parameters:
   i. Level of activation of a family helicase MDA-5;
   ii. The level of NOXA expression;
   iii. Or a combination thereof
c) Comparing the data obtained in step b) with those observed in control of the same cells treated similarly, but in the absence of the candidate compound;
d) Selecting the compounds who have given rise to a significant increase in the parameter or parameters determined in step b) in comparison with the control.

In this case the cell line would be selected from the group of pancreas cancer cell lines: IMIMPC2, MiaPaCa2, Aspc1, A6L, SKPC1 and Panc-1; or from the group of colon cancer cell lines: CACO, SW480 and SW1222; or from the group of bladder cancer cell lines: RT112, MGHU4, 639V, 253J, MGHu3 and SW1170; or from the group of glioma cell lines: U87MG, U251 and T98G; or from the group of breast cancer cell lines: MDA231, MCF7 and T47D; or from the group of prostate cancer cell lines: LNCaP, PC3 and DU145; or from the group of lung cancer cell lines: H1299 and NCI H460; or from the group of ovarian cancer cells lines: NCI H23, CHQK1 and SK-OV-3.

A preferred way of carrying out the process of the invention is described below, in the examples of the invention. In such a case, the process of the invention is performed by using a combination of MDA-5 activation determination, gene expression analyses (observing increases in NOXA expression) and confirmation of autophagy activation by the three possible methodologies already mentioned: monitoring of LC3 protein posttranslational modifications by immunoblot, track changes in the cellular distribution of LC3 by fluorescence detection due to the fluorescent protein GFP (with cells previously transfected with a recombinant retrovirus containing a structure capable of expressing the fusion protein GFP-LC3), confirmation of autophagosomes formation by electronic transmission microscopy at 5 hours of treatment with the candidate compound, and confirmation of phagocytic vacuoles at 30 hours of treatment.

Of note, the above explained process of the invention allowed the identification of a new compound comprising a combination of double-stranded RNA (dsRNA), or an analogue thereof, and a polycation. In a preferred embodiment of the invention, said compound is B0-110 (pIC^{PEI}), which consists of a combination of polyinosine-polycytidylic acid (pIC) which is at least 1000 nucleotides per chain in length and polyethyleneimine (PEI).

As demonstrated below, in the examples and figures of the present invention, BO-110 can be efficiently used for treating different types of cancer, for example: melanoma, pancreas, colon, bladder, breast, prostate, lung and ovarian carcinoma.

Therefore, the present invention also relates to a pharmaceutical composition comprising BO-110, for use in the treatment of cancer, for example: melanoma, pancreas, colon, bladder, breast, prostate, lung and ovarian carcinoma. This pharmaceutical composition is also useful for treating immunocompromised patients.

Surprisingly, the functional interaction of pIC and PEI achieves a synergistic technical effect, which improves and modifies the sum of the technical effects of the individual features. Thus, BO-110 enters cells and acts in a different manner than its components PEI and pIC. Specifically, while PEI has no measurable cellular effect, and the isolated pIC signals transiently induce immunoresponses which ultimately have no biological impact *in vivo*, BO-110 is able to selectively kill tumor cells. Therefore, BO-110 illustrates the concept of synthetic lethality described for genes or compounds, which as single agents have no activity, but that in combination have a different, and therapeutically exploitable anticancer effect.

Therefore, one of the most important points of the present invention is the unexpectedly discovery that the mimetic of viral dsRNA polyinosine-polycytidylic acid (pIC) changes its route of entry and delivery into the tumor cells. From a standard recognition by the TLR-3 (Toll like receptor 3), pIC can be targeted to a family of dsRNA sensors (different from TLR3), when this dsRNA is combined with a family of carriers that specifically allow for cytosolic delivery. This activity changes the mode of action of dsRNA from an inconsequential immunomodulator, to a massive killer of tumor cells. Anticancer activity was demonstrated with polyethyleneimine (PEI) as well as lipofectamine, polyfect or superfect. Although these carriers, on their own, were not biologically active as therapeutic agents, the present invention shows that they are able to protect the molecule of pIC, maintaining it in a stable form that permits the autophagy activation. Therefore, the combination of dsRNA/polycation, exemplified by BO-110, represents a new molecular entity with anticancer efficacy. More importantly, the mode of action of BO-110 was unanticipated. This compound promotes a dual induction of autophagy and apoptosis leading to a coordinated and selective killing of tumor cells, particularly but not exclusively to melanoma cells, without affecting the viability of normal compartments. The apoptotic machinery was engaged via the protein NOXA. Different to other NOXA-inducing chemotherapeutic agents, BO-110 does not require the tumor suppressor protein p53. This is an important advantage as p53 is mutated, deleted or inactivated in a vast majority of human cancers. The effect is clearly superior to the classical responses to naked viral RNA, which explains the poor results in the clinical studies of naked pIC for the treatment of melanoma.

Thus, BO-110, but not uncomplexted dsRNA, was sufficient to promote self- killing of cancer cells and block cancer metastasis *in vivo*, even in immunocompromised mice. The genetic, functional and ultrastructural analyses described later in this invention demonstrate that the induction of autophagy is not triggered by pIC for cell protection, but to selectively destroy tumoral cells. Further attesting to these results, although pIC was considered as an inducer of immunity controlled by IFN, the observed effect is independent on the activation of the pathway for production and secretion of IFN-α. Consistent with this observation, the autophagic pathway activation occurs even in immunocompromised animals. Altogether, these data demonstrate that BO-110 targets and identifies new points of intervention for clinical exploitation of intrinsic pathogen recognition programs, autophagy and tumor cell death.

Genetic and functional studies identified the endogenous MDA-5 as the link between the autophagic and the apoptotic pathway driven by BO-110. This is also different from previous disclosures with respect to MDA-5 that were restricted to apoptotic cell death by exogenous components. The MDA-5/NOXA interplay was also novel.

Thus, MDA-5 is presented as a suitable therapeutic target for the screening of agents for the treatment of cancer with the specific feature of triggering tumor self-destruction by auto/lysosomal and intrinsic apoptotic proteases. As mentioned before, this strategy has advantages over standard therapeutic agents that engage either of these mechanisms independently.

Similarly, an entity which has enabled the discovery of this pathway because is able to activate it, is the complex BO-110 or other combinations of an analogue of the dsRNA and a cationic carrier. These agents are therefore, good candidates to be used for manufacture of medicines for the treatment of cancer.

As used in the invention, the term "long fragment of double-stranded RNA" is used as opposed to fragments of RNA known as short RNAs or interfering RNAs (siRNAs). Therefore, it is considered that a double stranded RNA (duplex) is "long" if the double stranded RNA fragment comprises at least 25 nucleotides per chain. It is preferred that the fragment used is similar in length to the double-stranded RNA intermediates that appear in cells during the cell cycle of most RNA viruses which appear to be the natural substrate of the helicase family of MDA-5, so that the double-stranded RNA of the invention is considered "long" especially if it contains at least 100 nucleotides per chain and, more particularly, if it contains at least 1000 nucleotides per chain.

Fragments of double-stranded RNA that occur in nature and could be useful for the use of the invention could be the double-stranded RNA intermediates that occur during the cell cycle of Paramyxovirus (such as the virus of Newcastle disease, NDV, Sendai virus (SDV)), Rhabdovirus (vesicular stomatitis virus (VSV)); flavovirus (hepatitus C virus (HCV)), ortomyxovirus (Influenza virus) and picornavirus (virus of the brain-myocarditis (EMCV).

As for the double-stranded RNA analogues, besides the polyinosinic-polycytidylic acid (pIC), it may be useful for the invention other dsRNA mimics: a) those whose skeleton is formed by a compound similar to the ribose, such as those based on LNA (locked nucleic acid: resistant to hydrolysis), morpholino and PNA (peptide nucleic acid), b) those in which at least one of the typical nitrogen bases of nucleotides of RNA have been replaced by analogs, which may also lead to different pairings of natural phenomena such as diaminopurina (which is paired with uracil by three hydrogen bonds), the pair xanthine / Diamine pyrimidine (where the form keto / keto of purine, xanthine, forms three hydrogen bonds with the amine / amine pyrimidine), or the pair isoguanine / isocitosine (where the form amine / keto of purine, the isoguanine, forms three hydrogen bonds with the pyrimidine keto-amine, the isocitosine).

As for the polycation carrier, are suitable for the purposes of the invention use all of those capable of altering the permeability of the plasma membrane and / or induce endocytosis by promoting the entry into the cell of double-stranded RNA or its analog, and releasing them to the cytosol, thereby increasing the activation of cytosolic sensors of double-stranded RNA, such as the helicase MDA-5. In addition to the polyethylenimineI (PEI) and Lipofectamine, under this definition are covered poly-L-lysine, polisilazane, polidihidroimidazolenium, polialilaminem and ethoxylated polyethylenimine (ePEI).

The invention is now illustrated in more detail below through examples and figures.

### BRIEF DESCRIPTION OF THE FIGURES

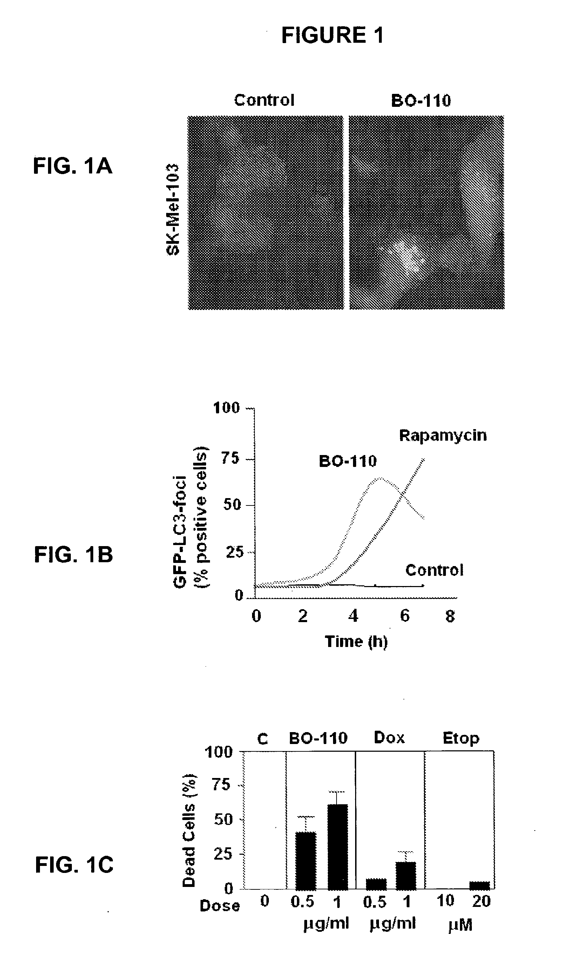

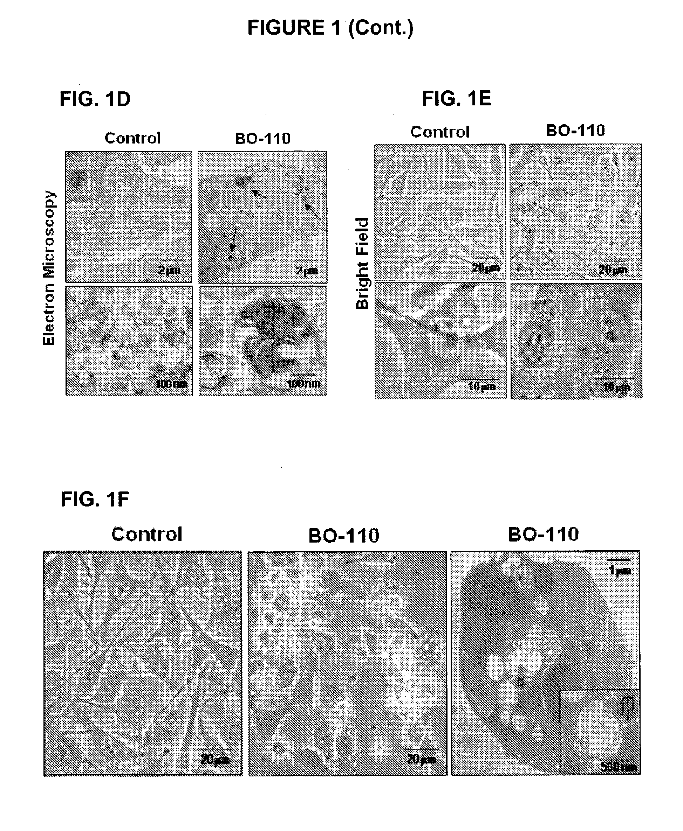

**Figure 1****. Induction of macroautophagy by BO-110 results in melanoma cell death.**
   Panel A shows the visualization by epifluorescence of autophagosome-like focal staining of eGFP-LC3 in SK-Mel-103 melanoma cells treated for 12 h with 1 µg/ml PEI-complexed pIC (BO-110). Cells treated with PEI as single agent are shown as reference controls.
   Panel B shows a time-dependent accumulation SK-Mel-103 cells showing punctuate fluorescence emission of eGFP-LC3 upon treatment with BO-110 or placebo control. Rapamycin was used as a classical autophagy inducer.
   Panel C shows immunoblots of total cell extracts isolated from SK-Mel-103 cells treated as indicated. Treatments are indicated in the top of each line: Control (no treated: cell populations incubated only in presence of vehicle), pIC or BO-110 treated cells. The proteins analyzed are indicated in the left side of the panels: non modified ATG8 (LC3 I), lipidated ATG8 (LC3 II) and loading control (β-actin). In the right side of the panel the Molecular Weights in kDa.
   Panel D shows electron microscopic micrographs of SK-Mel-103 cells treated with BO-110 or PEI control. Arrows point to membrane-bound autophagosomes and autolysosomes.
   Panel E shows microphotographs at high (top) and low (bottom) magnification of SK-Mel-103 cells 5 hours after incubation with vehicle (left side column) or BO-110 (right side column).
   Panel F shows representative microphotographs of bright field (left and center) and electron microscopy (right) of cell colonies after 30 hours of treatment as indicated in the pictures.

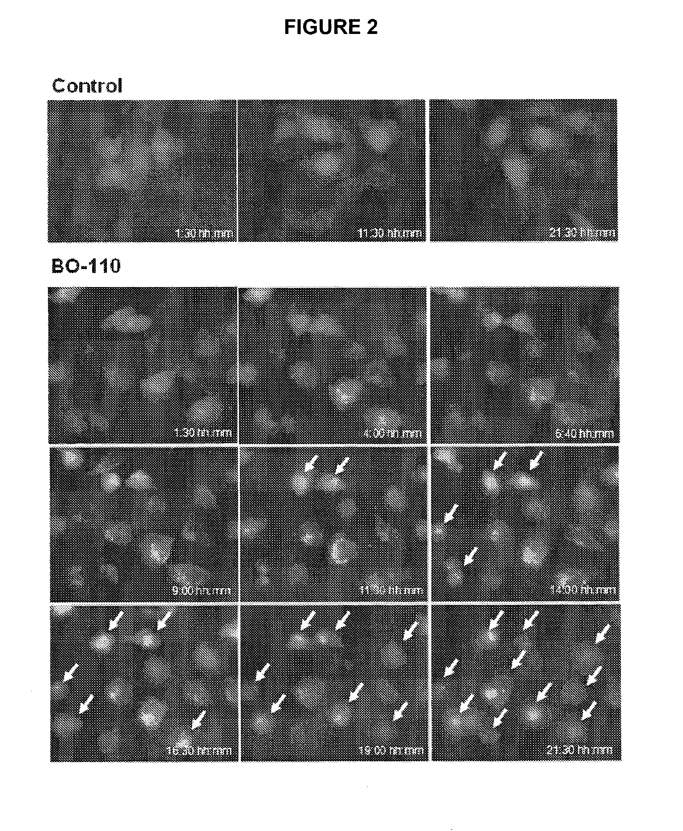

**Figure 2****. Microscopy at time intervals of autophagy induction by BO-110 in melanoma cells.**
   Sequence of microphotographs taken at the indicated time intervals after treatment with PEI (control) or BO-110 of eGFP-LC3 expressing SK-Mel-103 melanoma cells. The focal aggregates are indicative of autophagosome formation. Arrows mark cells collapsing and detaching (dying cells) during the treatment.

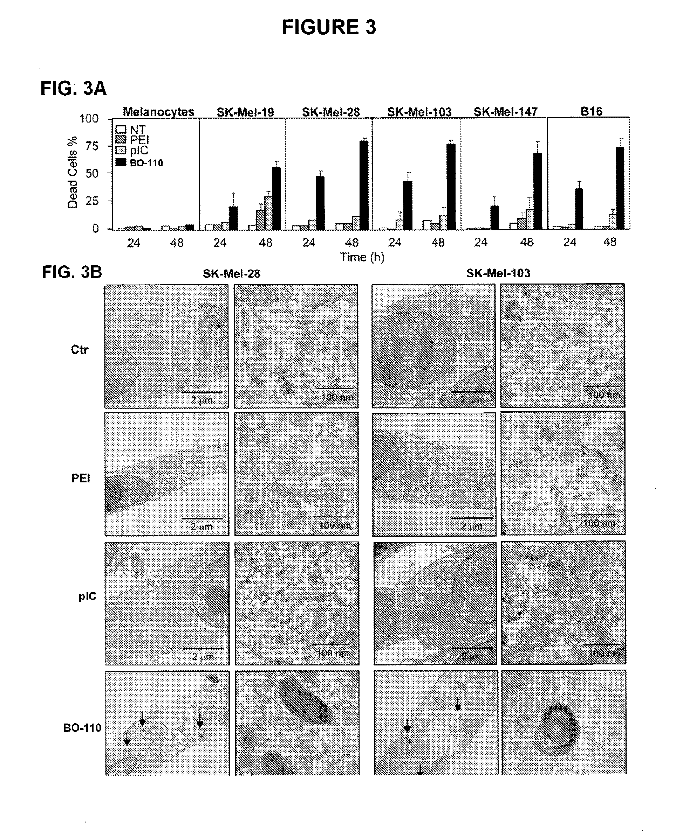

**Figure 3****. The delivery to the cytosol of pIC due to the presence of PEI triggers melanoma cells death in a selective manner.**
   Panel A shows the graphs where the percentage of cell death estimated by trypan blue exclusion assay is represented after 24 and 48 hours of treatment as indicated (NT: white bars, PEI treatment: dark grey bars pIC treatment: light grey bars, BO-110 black bars). Data are represented as mean ± SEM values of three independent experiments with the different cell lines indicated above the graphs. As shown. Only melanoma cell populations treated with BO-110 die efficiently.
   Panel B: electron microscope micrographs of SK-Mel-28 and SK-Mel-103 cells treated with vehicle (Ctr), PEI, pIC or BO-110, and visualized 12 h after treatment. For each cell line photographs taken with two different magnifications are shown. Arrows mark autolysosomes observed only in BO-110 treated cells.

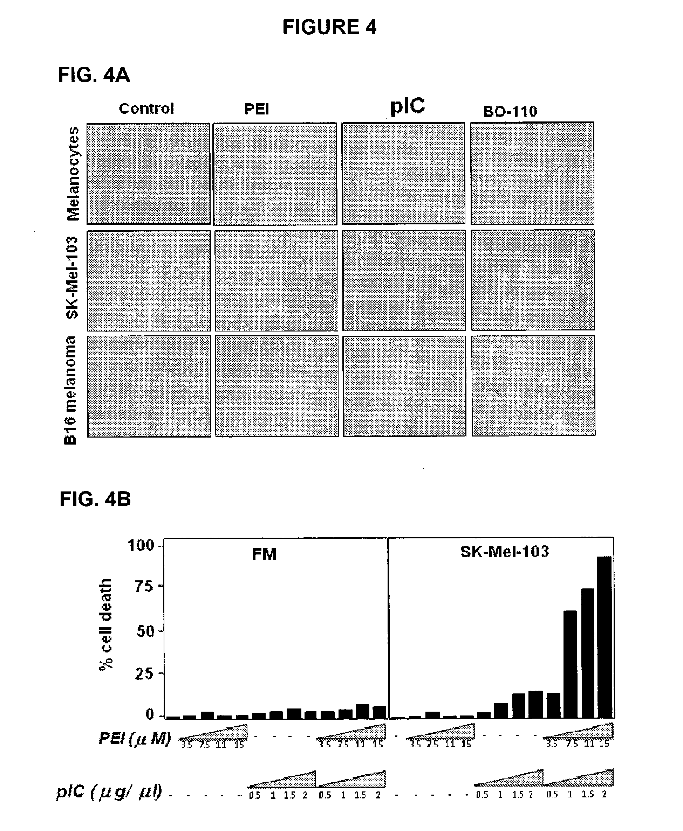

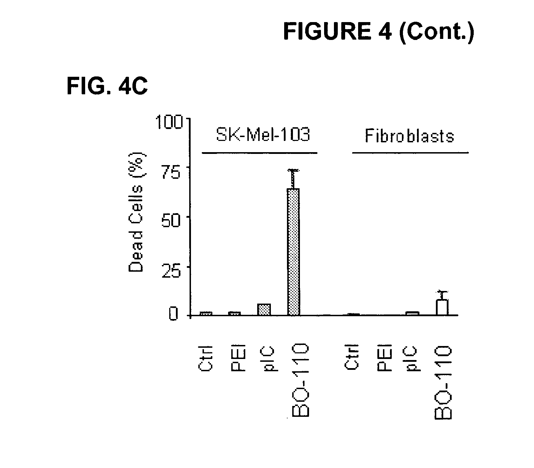

**Figure 4****. Selective citotoxic activity of BO-110 on tumoral cells.**
   Panel A shows representative bright field images of melanocytes isolated from human foreskin (upper row), human melanoma cells SK-Mel-103 (middle row) and murine B16 melanoma line (bottom row) after treatment with vehicle, PEI, pIC or BO-110, as indicated.
   Panel B shows dose-response curves to the PEI and pIC treatments as individual agents or in combination (BO-110) (right side bar groups in each graph), of FM (*foreskin melanocytes*) and SK-Mel-103. The response is expressed as percentage of dead cells 24 hours after treatment.
   The graph in panel C represents the percentage of cell death, estimated by trypan blue exclusion assays 24 hours after the treatments were performed (Ctrl: no treatment; PEI, pIC, and BO-110). The data are represented as means ± SEM of three independent experiments performed with the cell lines indicated (SK-Mel-103 or foreskin fibroblasts). As in panel A, it is shown that only melanoma cell populations treated with BO-110 die efficiently.

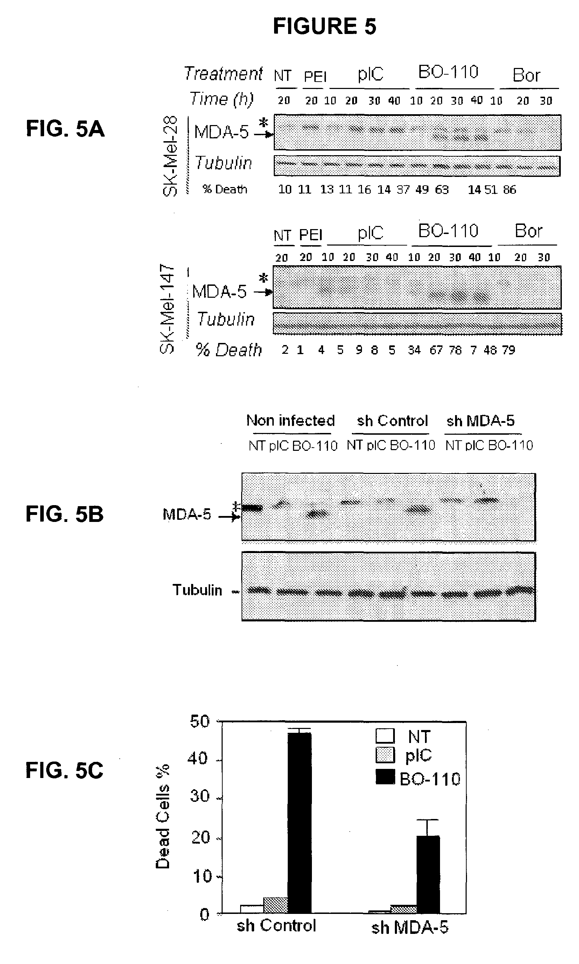

**Figure 5****. MDA-5 is a sensor and driver of BO-110 cytotoxicity in Melanoma Cells.**
   Panel A shows the immunoblots of total cell extracts isolated from SK-Mel-28 (upper photograph) y SK-Mel-147 (bottom) non tretaed (NT) or treated with PEI, pIC, BO-110 or bortezomib (Bor) as indicated in the different lines. Asterisk corresponds to a nonspecific band. Arrows indicate the position where it should be observed the 30 kDa band indicative of MDA-5 cleavage.
   Panel B: Processing of MDA-5 in SK-Mel-147 cells non infected or infected with lentivirus expressing scrambled or MDA-5 shRNAs visualized through immunoblotting of cell extracts after treatment with pIC, BO-110 or vehicle as indicated. As shown in panel A, asterisks mark a nonspecific band and arrows indicate the position of the 30 kDa band indicative of MDA-5 cleavage.
   Panel C shows the inhibitory effect of MDA-5 shRNA on the targetted toxicity induced by BO-110 addresed by trypan blue exclusion assays 24 hours after treatments pIC alone (grey bars, 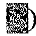 ), BO-110^{I} complex (black bars, 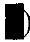 ) or no treated cells (non filled bars). Results from infection with control shRNA ("sh Control") are also shown. Data are represented as means ± SEM of three independent experiments.

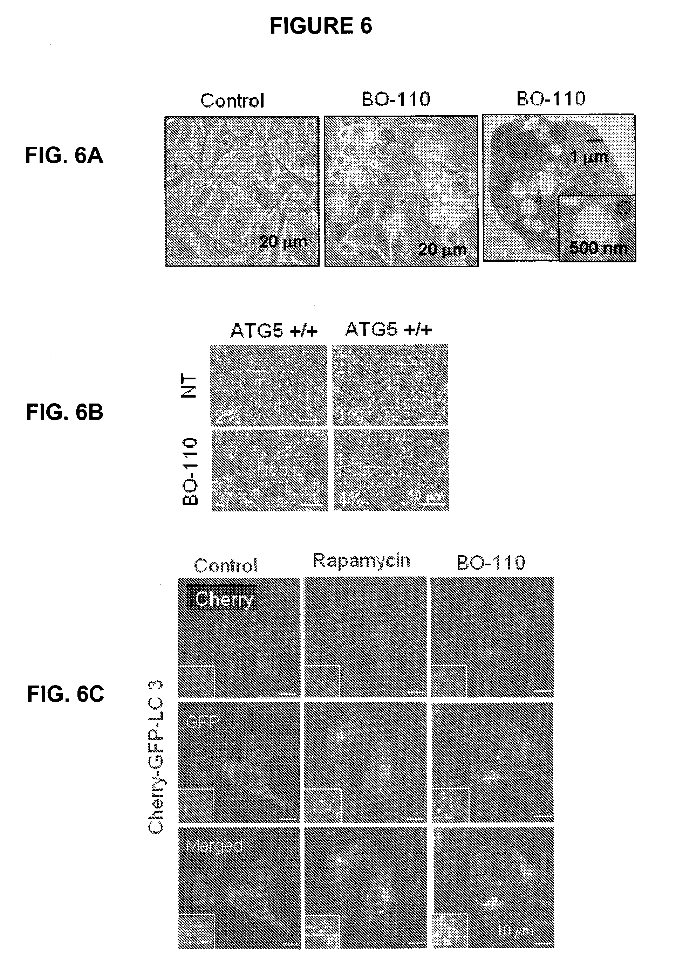

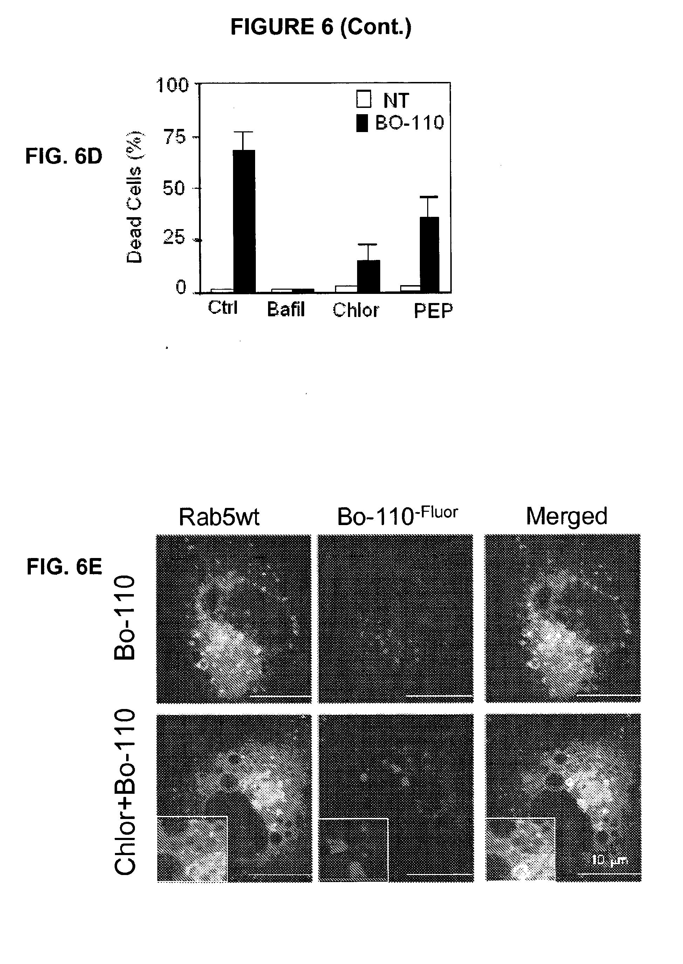

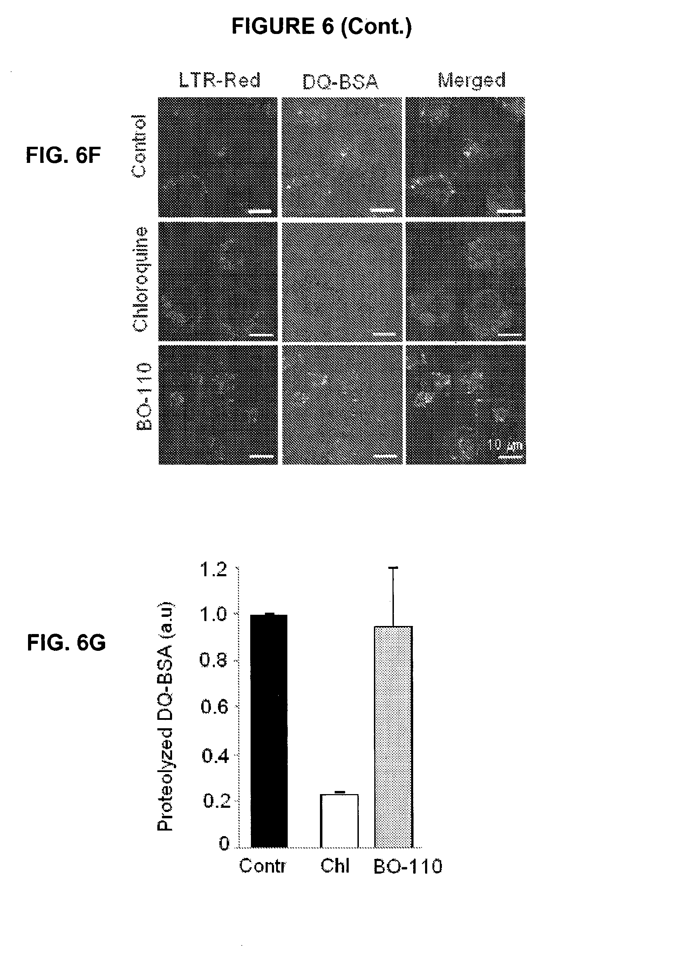

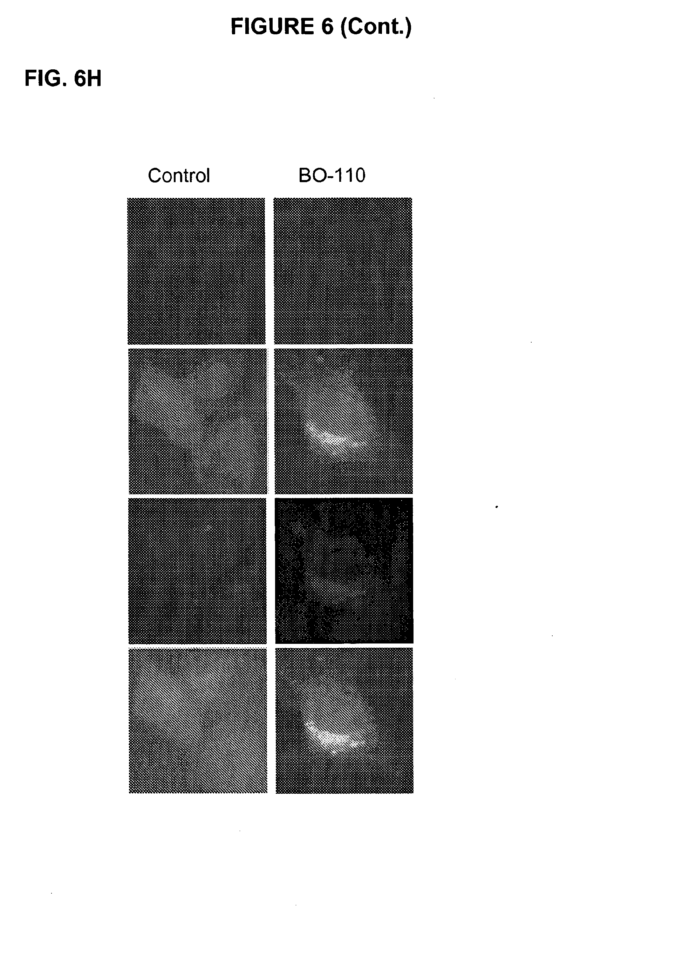

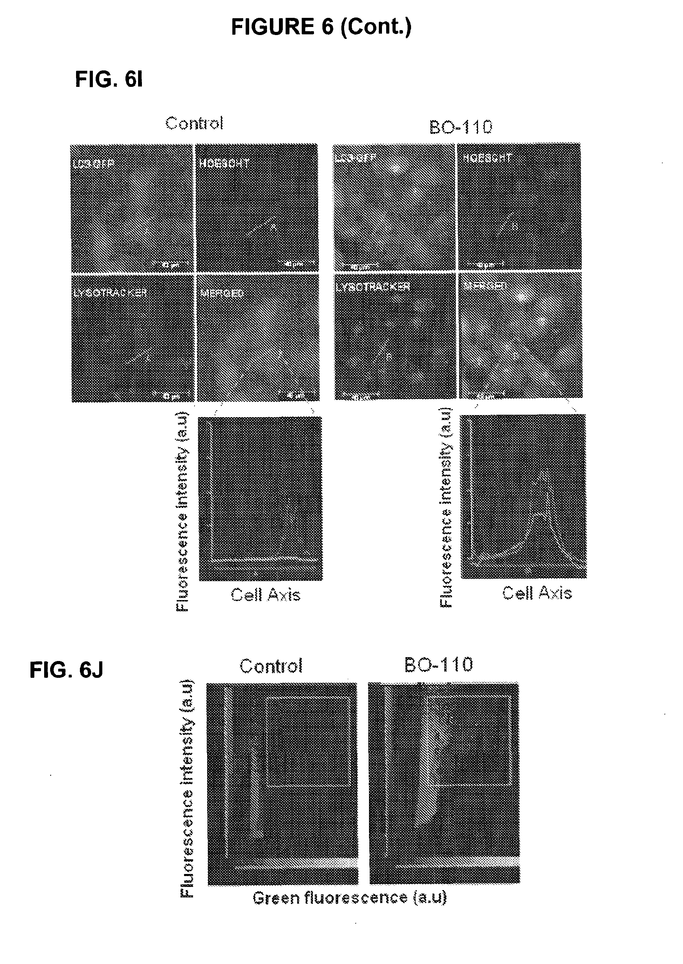

**Figure 6****. Effect of autophagy pharmacological inhibitors on the BO-110 cytotoxic activity.**
   Panel A shows the effect of 3-methyladenine (3-MA) and chloroquine (Chlor) on the EGFP-LC3 relocation in the autophagosomes, evaluated from the percentage of SK-Mel-103 cells that had fluorescence foci due to GFP-LC3 12 h after treatment with BO-110 (black filled bars) or with buffer control (unfilled bars).
   The panel B shows the inhibitory effect of chloroquine (Chlor), pepstatin A (PEP) or E64d on cell death estimated by trypan blue exclusion 20 hours after treatment with vehicle (white bars) or BO-110 (black bars). Data are shown as mean ± SEM of three independent experiments
   Panel C shows fluorescence confocal micrographs of SK-Mel-103 cells transfected with Cherry-GFP-LC3 to detect the autophagosomes formation (35 red and green foci) and autolisosomes (just red foci) after treatment with BO-110 or 25 nM rapamicine.
   Panel D shows the inhibitory effect of 100 µM 100 bafilomicine (Bafil), 20 µM Chloroquine (Chlor) or 10 µg/ml pepstatin (PEP) on cell death estimated by trypan blue exclusion 20 hours after treatment with vehicle (white bars) or BO-110 (black bars) Data are shown as mean SEM of three independent experiments
   Panel E shows confocal fluorescence images of SK-Mel-103 cells treated with BO-110 (top photo) or with BO-110 in the presence of chloroquine (bottom row of photographs) and stably transfected with EGFP-Rab5 wild-type (in the figure, column on the left) or incubated with BO-110 Red Fluor-labeled (column middle photographs). Internalization BO-110 in melanoma cells can be observed in the absence or presence of chloroquine.
   Panel F shows confocal fluorescence images for viewing lysosome-dependent proteolysis by the existence of cleavage and release of fluorescent DQ-BSA (resulting in green fluorescence in both SK-Mel-103 cells treated with vehicle (Control) and with BO-110. In the presence of chloroquine (Chlor: middle row of pictures), no fluorescence signal is observed in the column of DQ-BSA (middle column). Simultaneous images of the cells in the presence of Lysotracker- Red (LTR-Red: left column of photographs) to display the lysosomal compartment, which gave rise to signal in all three rows of photographs.
   Panel G shows a graph bar which represents the colocalization of the corresponding to DQ-BSA and Red-Lysotracker signals in the test cells of panel F (Ctrl: control, black filled bars, 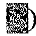 ;Chl: chloroquine, unfilled bars; BO-110, grey-filled bars, 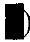 ). The colocalization is estimated in a minimum of 150 cells in two independent experiments and expressed with respect to the value obtained in control cells (AU: fluorescence arbitrary units).
   Panel H shows confocal immunofluorescence images of fluorescence foci due to GFP-LC3 (green in the original signal, indicative location of autophagosomes) and Lysotracker (red in the original signal, indicative of the presence lysosomes) in SK-mel-103 after treatment with BO-110 or buffer control, as indicated in the images. The nuclei were stained with Hoescht (Photo above, with blue signal in the original). In the bottom row the superposition of the three previous images are shown, which give a yellow or orange color in areas that had green and red signal, images corresponding to GFP-LC3 and Lysotracker respectively, indicating that signals corresponding to GFP-LC3 and Lysotracker are located in the same areas.
   Panel I shows confocal microscopy images taken in real time of SK-Mel-103 cells expressing EGFP-LC3 (green signal in the original) treated either with buffer control with pIC ("Control") or with BO-110 and incubated in the presence of Lysotracker (red signal in the original) or Hoescht (blue signal in the original) as shown on the images. The superposition of the three images (Bottom right of each treatment, control or BO-110), revealed a strong colocalization (yellow signal) between GFP-LC3 and lysosomes (as expected by the formation of autolisosomes) after treatment with BO-110 but not after treatment with naked pIC. In the third row of the panel, under the result of overlapping images, it is shown the graphs obtained by quantifying the total cell fluorescence intensity in the green channel in a given plane (Graph labeled "GFP" for EGFP-LC3) and red (marked graph as "LYSO" for the Lysotracker). In the case of the graphs obtained with cells treated with BO-110, the similar distribution of both signals, eGFPLC3 and Lysotracker, is indicative of colocalization and therefore the fusion of autophagosomes and lysosomes.
   Panel J shows a representation of a population-based analysis of SK-Mel-103 treated cells with pIC (Control) or with BO-110, which represents the signal intensity of EGFP-LC3 (green fluorescence, x-axis) and Lysotracker (Signal red, Y axis). The squares include cells with dual staining of both markers.

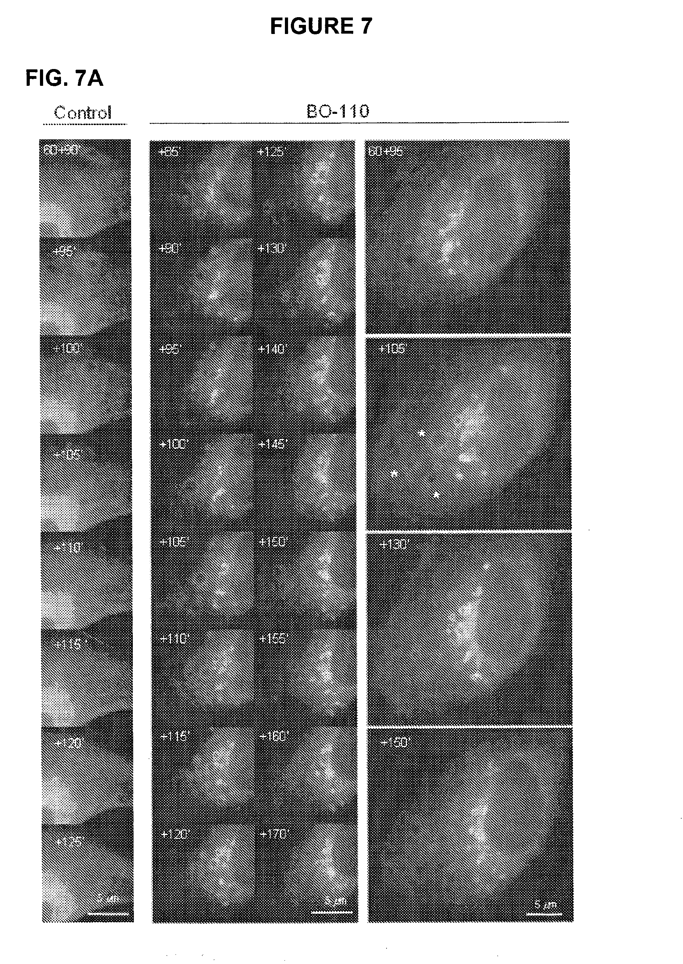

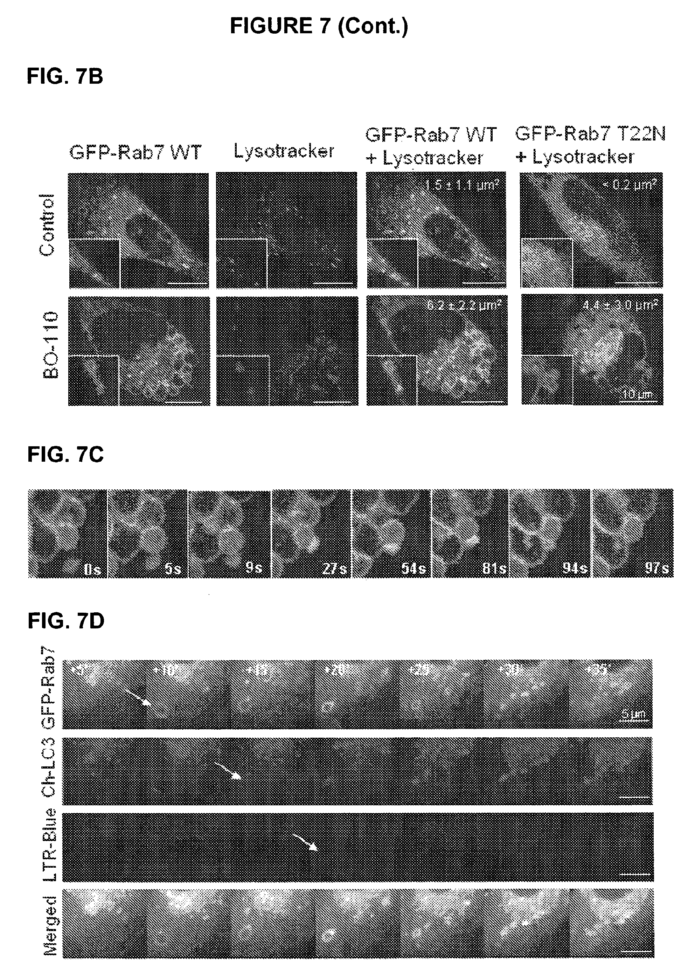

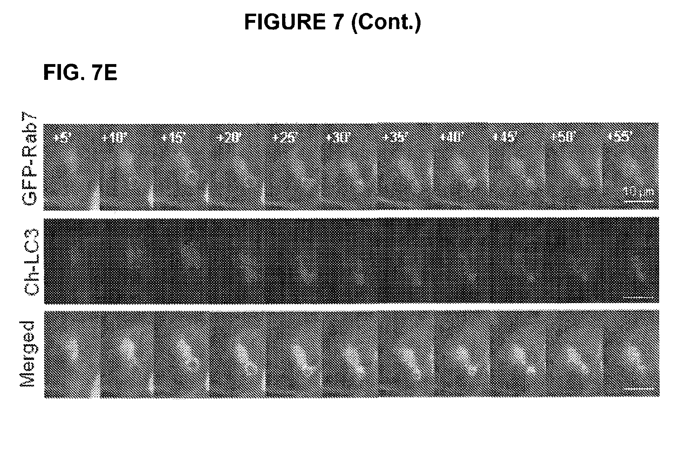

**Figure 7****. Endosomal traffic and generation and resolution of amphisomes upon BO-110 treatment.**
   Panel A shows sequential images of SK-Mel-103 melanoma cells expressing EGFP-Rab7, captured by real time fluorescence microscopy, at the indicated times after treatment with BO-110 or vehicle control. Of note, BO-110 resulted in the generation of a large number of vesicles. The asterisks mark the endosome-endosome fusions (for clarity, are shown only some examples.)
   Panel B shows confocal microscopy photographs of SK-Mel-103 cells stably transfected with retrovirus which resulted in the expression of green fluorescence by EGFP-Rab7 wt fusion protein (wild-type Rab7) (first and third column of photographs from the left, in the second case the picture was obtained in the presence of Lysotracker-Red, as indicated on the column) or the fusion EGFP-Rab7 T22N incubated in the presence of Lysotracker (Right column of photographs). The cells were treated additionally with BO-110 (bottom row of panel) or with the vehicle control (top panel). Images were captured 10 hours after treatment with BO-110. The two columns of photographs located on the right contain values corresponding to the average area contained in Rab7 decorated vesicles.
   Panel C displays a sequence of confocal micrographs taken in the indicated time intervals (in seconds) shown on the photographs, which illustrate the fusion and incorporation of lysosomes to Rab7-positive vesicles after treatment with BO-110.
   Panel D shows real-time fluorescence microscopy images treated SK-Mel-103 with BO-110 and stable transfected with retrovirus that gave rise to green or red fluorescence, as give it by the GFP-Rab7wt (GFP-Rab7 in the photographs), Cherry-LC3 (Ch-LC3 in the photographs), or blue fluorescence due to the Lysotracker-Blue (LTR-Blue in the Figure). The images were taken in the indicated times (minutes) 1 hour after the treatment. The arrows mark the first sequence in which each marker indicated were able to be visualized.
   Panel E shows the incorporation of LC3 on the surface of the vesicles with endosomal Rab7 prior to its internalization and subsequent degradation. These endosome/LC3 hybrid structures (amphysome) were visualized by real time microscopy fluorescence of cells SK-Mel-103 expressing EGFP-Rab7 or Cherry-LC3 (Ch-LC3 in the photographs).

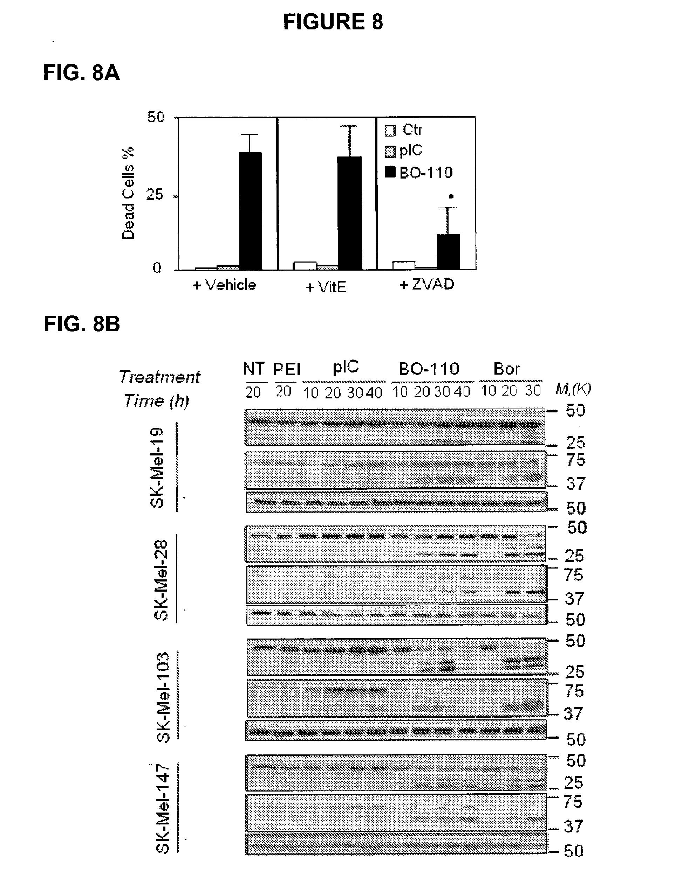

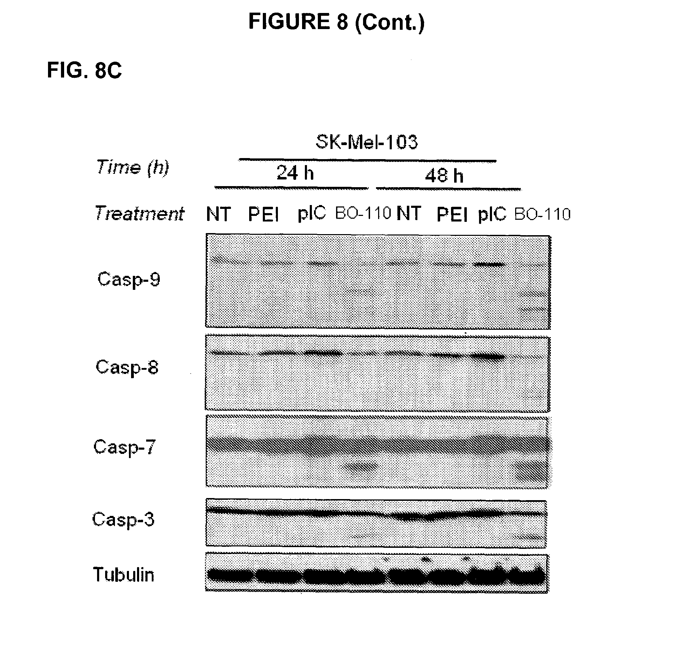

**Figure 8****. BO-110 cytotoxicity is dependent on the activation of effector and regulatory caspases.**
   Panel A shows the percentage of cell death caused by treatment of SK-Mel-147 cells with PEI as buffer control (Ctr, represented by unfilled bars), pIC (gray-filled bars) or the complex BO-110 (black filled bars) in the presence of the compounds listed under each of the graphs: the vehicle (control buffer without PEI) (left graph), vitamin E (+ Vite, middle graph) or the caspase inhibitor ZVAD-fmk (+ ZVAD, graphic, right). The percentage was measured in all cases with trypan blue exclusion.
   Panel B shows the results of an immunoblot of metastatic melanoma cell lines extracts (indicated on the left side). They were obtained by collecting the cells in the indicated post-treatment times (in hours, on each lane). Treatments are indicated on the times: NT (no treatment: control cell populations incubated in the presence of buffer), PEI, pIC, complex BO-110 or Bor (bortezomib 25 mM). Next to each picture the protein analyzed is shown: casp-9 (caspase 9), casp-8 (caspase 8) or tubulin (loading control). The numbers on the right side indicate the relative mass in kDa, corresponding to the protein bands present at that height.
   Panel C shows the results of an immunoblot of SK-Mel-103 cell line extracts, obtained by collecting the cells after treatment indicated in hours (24 and 48 hours). Treatments are indicated on the rails, under time: NT (no treatment: control cell populations incubated in the presence of only buffer), PEI, pIC and complex BO-110. Next to each picture the protein analyzed is shown: casp-9 (caspase 9), casp-8 (Caspase 8), casp-7 (caspase 7), Casp-3 (caspase 3) or tubulin (loading control).

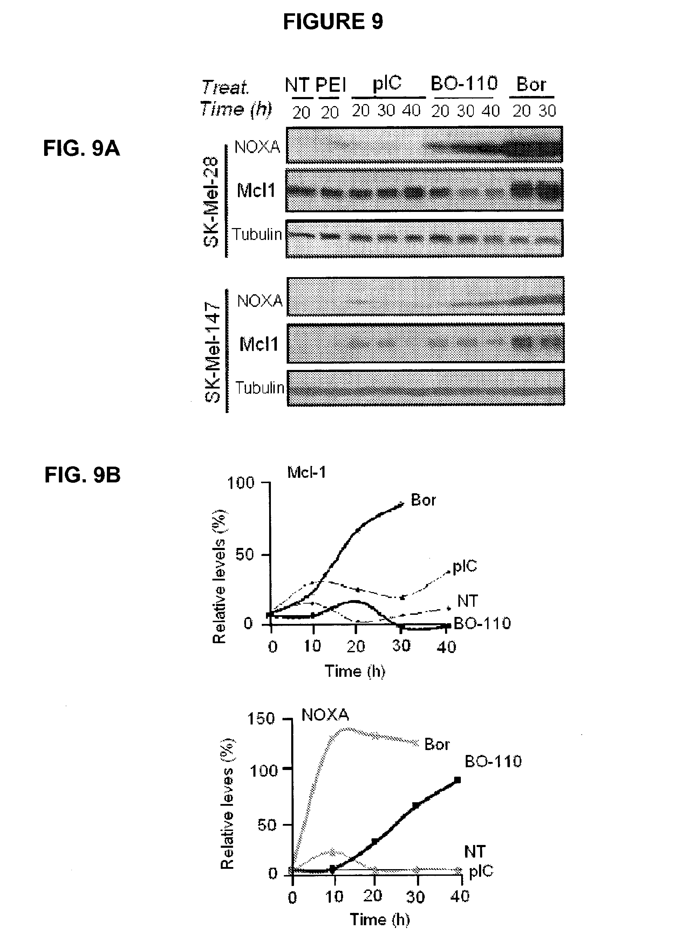

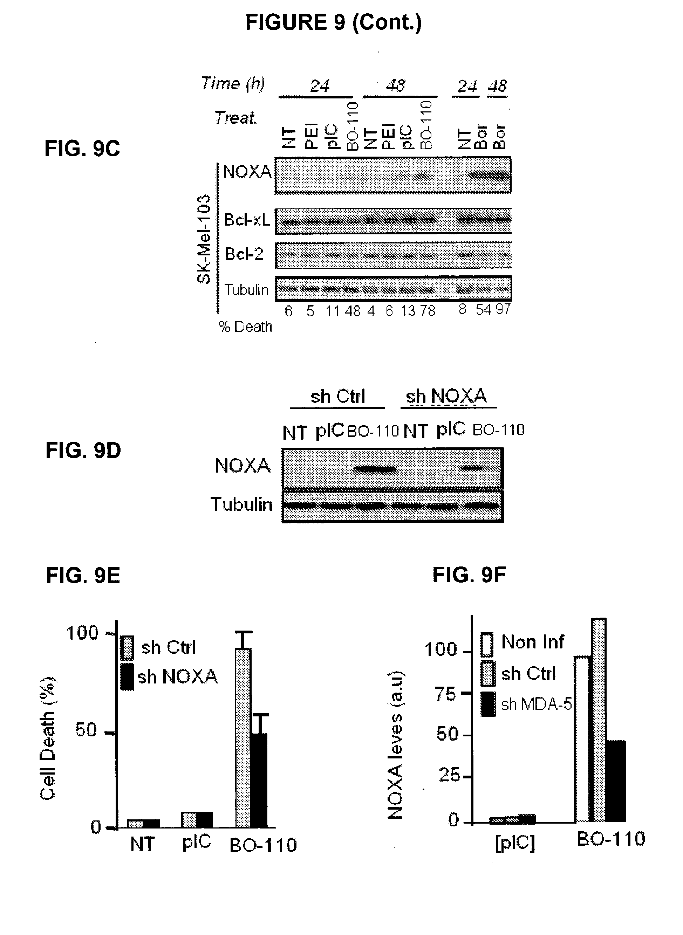

**Figure. 9** **BO-110 triggers apoptosis via NOXA independent of p53 status and without inducing compensatory activation of MCL-1.**
   Panel A shows photographs of immunoblots from SK-Mel-28 cells (above) or SK-Mel-147 (lower) obtained by collecting cells at the times indicated after treatment (in hours). Treatments are indicated on the times: NT (no treatment: control cell populations incubated in the presence of buffer), PEI, pIC, complex BO-110 or Bor (bortezomib 25 mM). Next to each picture the protein whose level was analyzed is shown: NOXA, Mcl-1 or tubulin (loading control).
   Panel B shows two separate graphs that represent the relative levels of Mcl-1 (top) and NOXA (lower graph) calculated by densitometry after immunoblotting obtained from SK-Mel-28 cells as a function of time since treatment is indicated, represented as the percentage referred to the corresponding value obtained in untreated control cells. Next to each curve the corresponding treatment is indicated.
   Panel C shows photographs taken from immunoblotting of SK-Mel-103 total cell extracts, obtained by collecting the cells after treatment (indicated times in hours) for each treatment group. Treatments are indicated on each lane: NT (no treatment: control cell populations incubated in the presence of buffer), PEI, pIC, complex BO-110 or Bor (bortezomib 25 mM). Next to each picture the protein whose level was analyzed is shown: NOXA, Bcl-xL, Bcl-2 or tubulin (loading control). In the bottom of the panel the percentage of cell death observed after treatment is indicated.
   Panel D shows pictures of immunoblots designed to evaluate the expression of NOXA protein Extracts were obtained from SK-Mel-103 melanoma cells treated for 24 h with pIC or BO-110 two days after infection with a lentiviral vector expressing an inactive shRNA (sh Ctrl) or a shRNA directed against NOXA.
   Panel E shows a graph which represent the death rates of SK-Mel-103 melanoma cells (expressed as a percentage of dead cells), either transduced with a control shRNA (gray-filled bars, 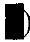 ) or a shRNA directed against NOXA (black filled bars, 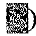 ) and incubated with pIC or BO-110 for 24 h (NT: no treatment, cells incubated with the vehicle of administration.)
   Panel F corresponds to the inhibitory effect of MDA-5 downregulation on the induction of NOXA by BO-110, represented by NOXA levels (expressed in arbitrary units, au) in SK-Mel- 103 cells transduced with shRNA control or a shRNA directed against MDA-5. NOXA levels were measured by densitometry and represented over untreated controls (N Inf: no interference, levels that given the value 1 in the case of treatment with naked pIC and 100 in the case of BO-110 treatment).

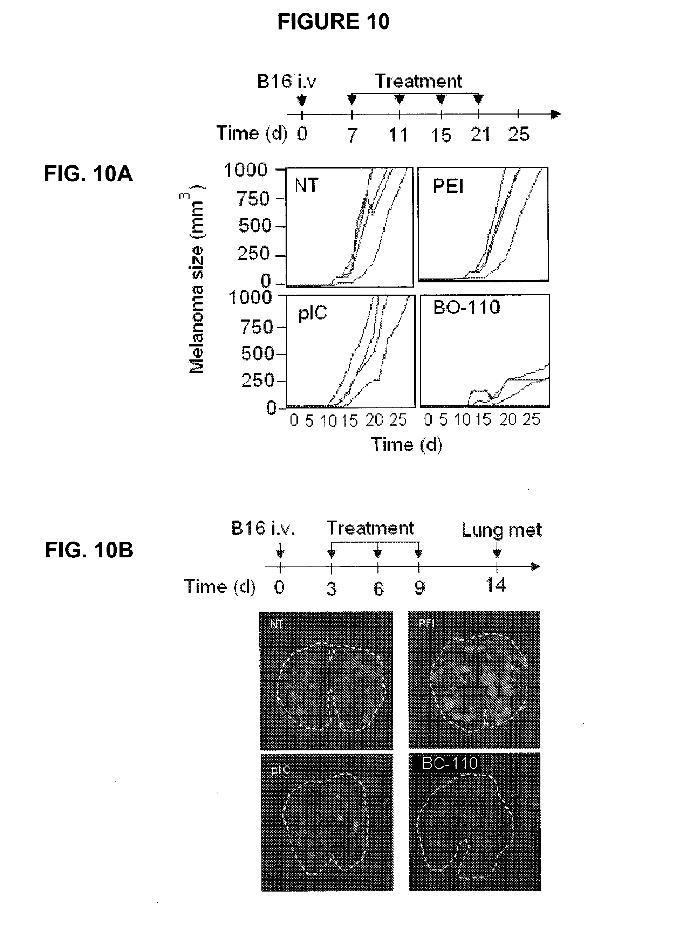

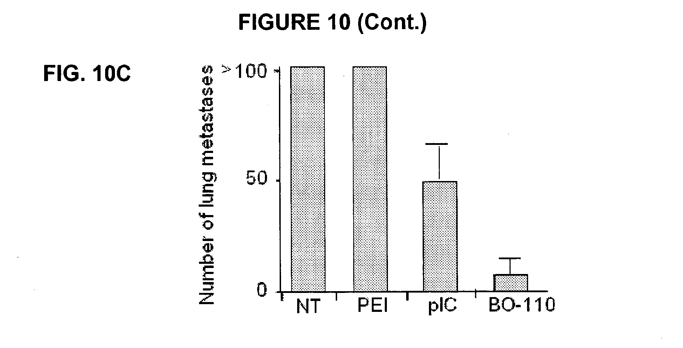

**Figure 10****. Anti-melanoma activity of BO-110 in immunocompetent mice.**
   Panel A.

      Upper panel. Schematic of the experimental approach to generate s.c. xenografts of B16 melanoma cells in syngeneic C57BL/6. Treatment times for peritumoral injections of 50 µg (in 100µl) (2 ng/kg) of naked and PEI-complexed pIC are also indicated. Control groups received 100 µl of 5 % glucose or only PEI.
      Bottom panel. Representation of tumor growth estimated by caliper measurements at the indicated time points. 10 tumors were analyzed per experimental group. Routinely, mice were sacrificed when the tumor volume exceeded 1000 mm³. The experiment was repeated twice with similar results.
   Panel B shows the intravenous implantation of B16-EGFP melanoma cells in syngeneic C57BL/6 for subsequent intravenous treatments with 10 µg (in 100µl) (1 ng/kg) of pIC or BO-110 at the indicated time points. Control groups received PEI in 5% glucose. 14 days after cell inoculation, mice were euthanized, and lungs processed for fluorescence imaging.
   Panel C shows a bar graph which represents the number of lung metastases observed in each treatment group obtained manual counting of external metastasis (C). (*P**<0.01 between NT/PEI and BO-110; *n*=5; generalized Mann-Whitney test).

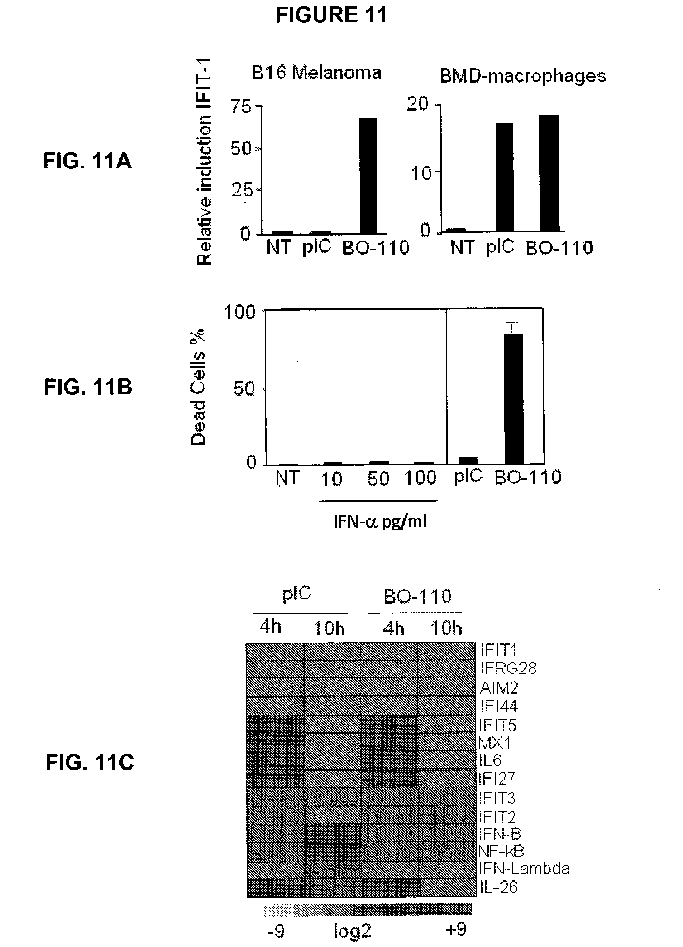

**Figure 11****. IFN-α is induced by BO-110 but is not sufficient to promote melanoma cell death.**
   Panel A shows B16 melanoma cells and bone-marrow-derived macrophages treated with pIC or BO-110, RNA was isolated and quantitative PCR was performed for the IFN target IFIT-1. Shown the relative mRNA levels of IFIT-1 estimated with respect to control untreated cells.
   Panel B shows SK-Mel-103 melanoma cell were treated with the indicated concentrations of human recombinant IFN-α starting from 10 pg/ml already higher the secreted amount of IFN-α in BO-110-treated cells determined by ELISA). Cell death was determined 24 h after treatment. As controls, cells were treated in parallel with BO-110 (24 h). Note that high levels of IFN-α are not cytotoxic to melanoma cells, and cannot recapitulate the efficient killing by BO-110.

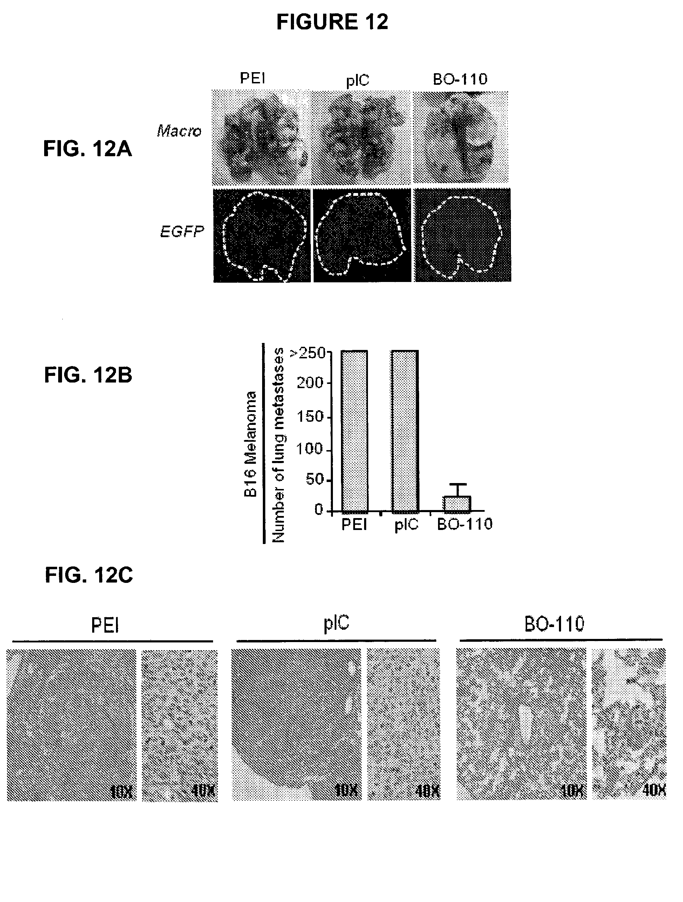

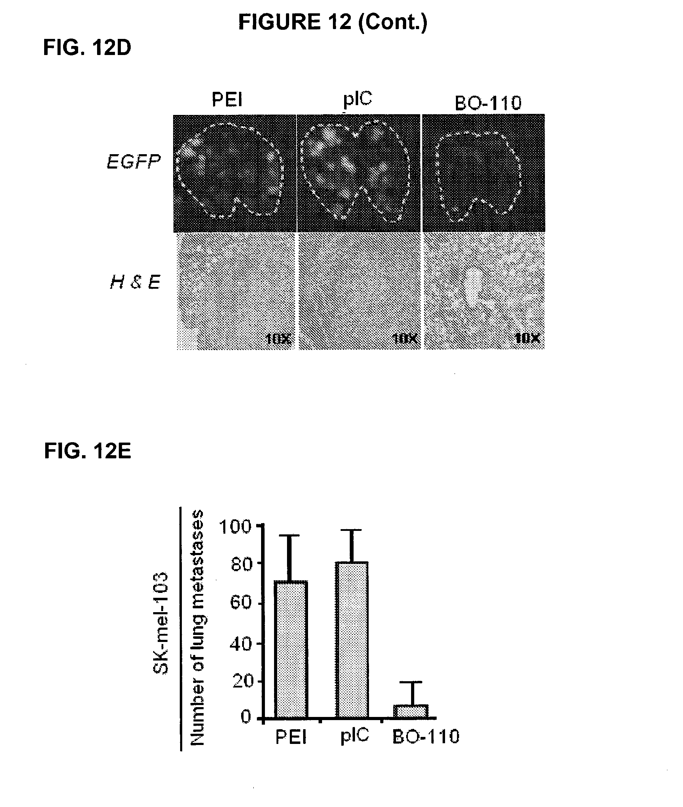

**Figure 12****. Immunosuppression does not compromise the ability of BO-110 to block metastatic dissemination of melanoma.**
   Panel A shows the generation and treatment of B16-driven melanoma lung metastasis in SCID Beige mice (severe immunedeficience for NK, B and T cells). Images correspond to photographs under visible or fluorescent light of representative lungs of mice inoculated i.v. with B16 melanoma cells, and treated with PEI, pIC or BO-110. Images were captured 14 days after cell injection.
   Panel B shows a representation of the mean number of metastases induced by B16 as indicated in panel A (*P**<0.01 between PEI, pIC and BO-110 treatment groups; *n*=5; generalized Mann-Whitney test).
   Panel C shows histological analysis of B16-driven lung in mice treated with PEI, pIC or BO-110. Shown are representative H&E stains of lungs from the indicated treatment groups and visualized at two different magnifications (10x and 40x).
   Panel A shows the generation and treatment of SK-Mel-103 -driven melanoma lung metastasis in SCID Beige mice (severe immunedeficience for NK, B and T cells). Images correspond to photographs under fluorescent light or visible H&E stains (lower line) of representative lungs of mice inoculated i.v. with SK-Mel-103 melanoma cells, and treated with PEI, pIC or BO-110.
   Panel E shows a representation of the mean number of metastases induced by SK-Mel-103 as indicated in panel D. (*P**<0.01; *n*=5; generalized Mann-Whitney test).

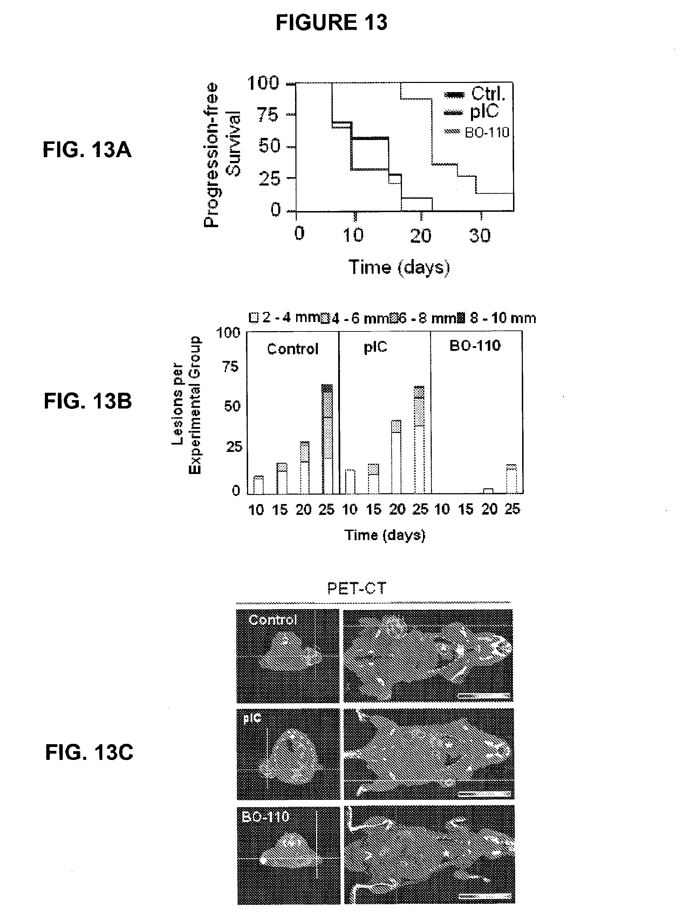

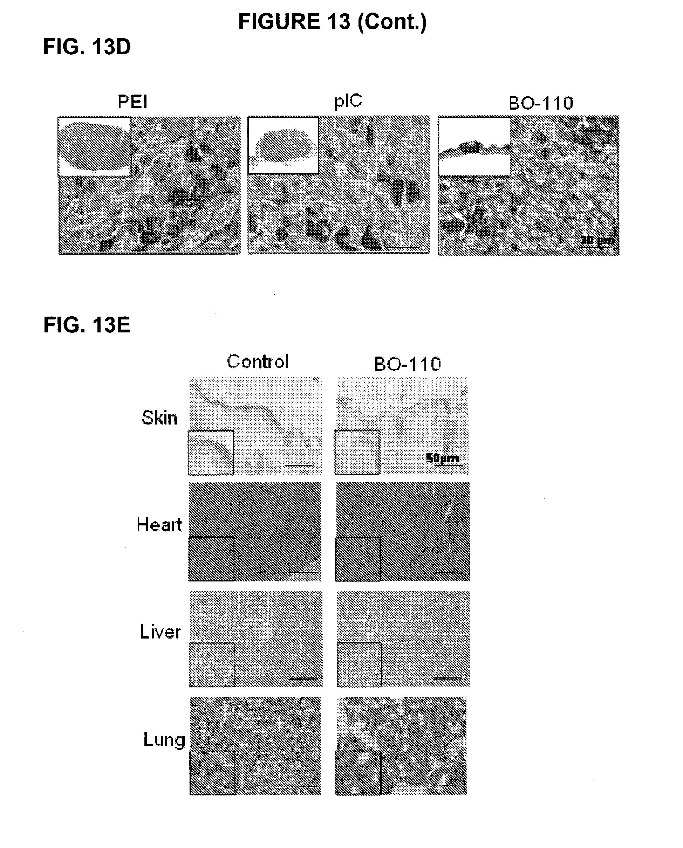

**Figure 13****. Inhibition of metastatic dissemination spread by BO-110 in Tyr::NRAS^{Q61K} x INK4a/ARF^{-/-} mice.**
   Panel A shows a Kaplan-Meier plot for progression-free survival of metastasis in Tyr:: Tyr::Ras^{Q16K} x INK4a/ARF^{-/-} mice treated with DMBA to induce pigmented lesions and then treated with PEI in 5% glucose (Control: Ctrl.) pIC or BO-110.
   Panel B shows bar graphs for the cumulative average number of cutaneous melanocytic neoplasms developed by each of the test groups of panel A. The count was performed every 5 days and the tumors were grouped by size ranges as indicated on the graphs.
   Panel C shows representative images of cross sections (left column) and coronal sections (right column) obtained by PET/CT aimed to test the metabolic activity (incorporation of 18F-FDG), of representative mice examples treated with PEI in 5% glucose (control), pIC naked or BO-110. The tumors are surrounded by dotted white lines. Asterisks indicate the position of the animal's hearts.
   Panel D shows hematoxylin - eosin staining of melanocytic lesions samples taken from each of the treatment groups described in panel A.
   Panel E shows hematoxylin - eosin staining of skin tissue samples, heart, liver or lung (as indicated on the left of the photographs) of mice treated with 5% glucose vehicle (Control) or with BO-110, which demonstrate the no toxicity associated with BO-110 treatment in normal cells compartments.

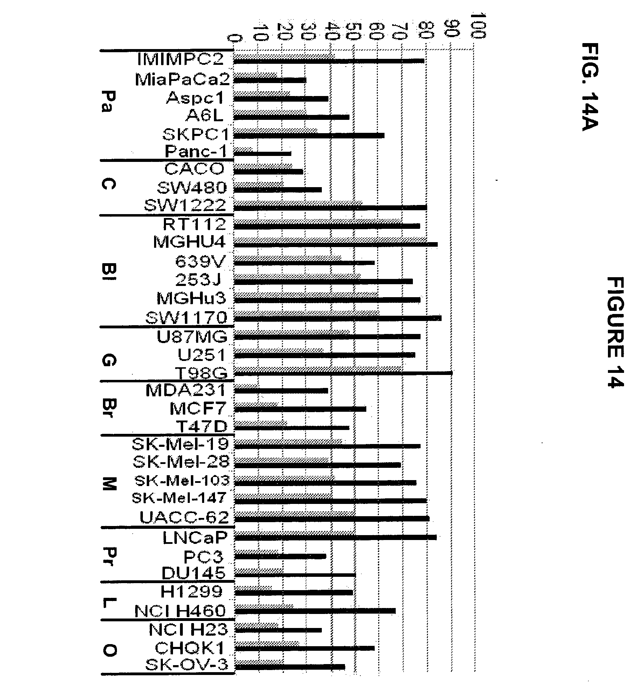

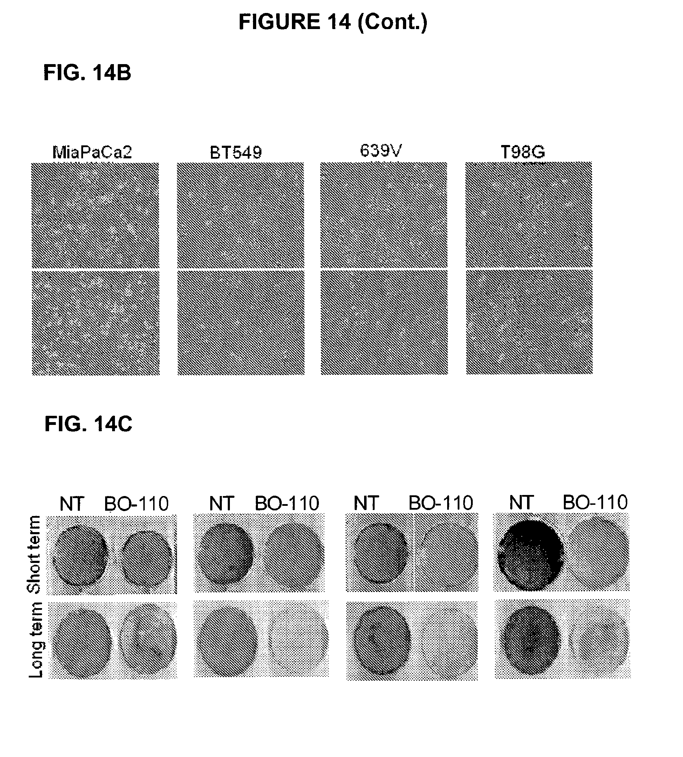

**Figure 14****. Citotoxic activity of BO-110 on a variety of tumor cells.**
   Panel A represents the percentage of cell death in different tumor cell lines: pancreas (Pa), colon (C), bladder (B1), glioma (G), breast (Br), melanoma (M), prostate (Pr), lung (L) and ovarian (O) carcinoma, estimated by trypan blue exclusion assays performed 18 hours (left bar) and 30 hours (right bar) after BO-110 treatment. Data are represented as means ± SEM of three independent experiments performed with the indicated cell lines. Panel B shows representative bright field images of the following tumor cell lines: MiaPaCa (pancreas), BT549 (breast), 639V (bladder) and T98G (glioblatoma) after 24 hours treatment with vehicle or BO-110, as indicated. Although BT549 was initially resistant to BO-110, it finally collapses in long term viability assays (see panel B). 639V and T98G are highly sensitive to the cytotoxic effect of BO-110.
   Panel C shows viability assays of the indicated tumor cell lines after 24h treatment with vehicle or BO-110. For short viability assay, treated cells were fixed 24 hours after treatment and stained with crystal violet for visualization of colony cell formation. For long term viability assay, one tenth of cells treated for 24 hours were plated and fixed and stained with crystal violet 48h later.

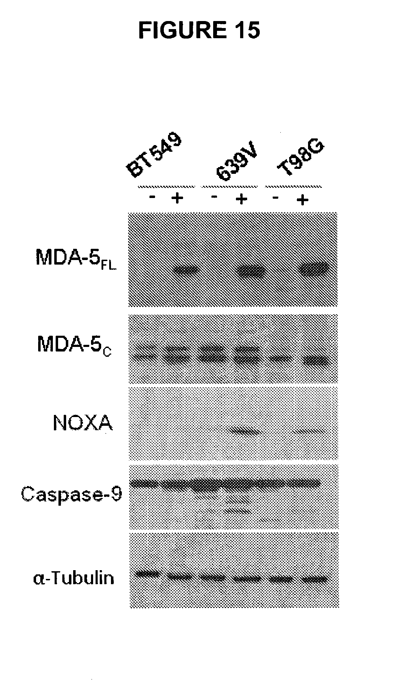

**Figure 15****. BO-110 induced cell death is dependent on the activation of MDA-5, Noxa and Autophagy in tumor cell lines.**

This figure shows immunoblots of total cell extracts isolated from the following tumor cell lines: BT549 (breast), 639V (bladder) and T98G (glioblastoma) after 24 hours treatment with vehicle (indicated as "-") or BO-110 (indicated as "+"). The proteins analyzed were: MDA-5_{FL} (MDA-5 full length), MDA-5_{C} (MDA-5 cleavage), NOXA, Caspase- 9 or tubulin (loading control). Note the higher induction of NOXA and MDA-5_{FL} and the cleavage of Caspase-9 and MDA-5_{C} occur in the more BO-110 sensitive tumor cells.

### EXAMPLES

The assays from the examples described below were carried out with the following materials and experimental techniques:

### Cells and Cell Culture.

The human metastatic melanoma cell lines SK-Mel-19, SK-Mel-28, SK-Mel-103 and SK-Mel-147 and the mouse B16 cells have been described before (Soengas et al. 2001. These cells were cultured in Dulbecco's modified Eagle's medium (Life Technologies, Rockville, MD, USA) supplemented with 10% fetal bovine serum (Nova-Tech Inc., Grand Island, NY, USA).

Human melanocytes were isolated from human neonatal foreskins as described (Fernández et al., 2005) and maintained in Medium 254 supplemented with melanocyte growth factors (HMG-1), containing 10 ng/ml phorbol 12-myristate 13-acetate (Cascade Biologics, Portland, OR, USA).

The human fibroblast were isolated from human neonatal foreskins and maintained in DMEM medium supplemented with 10% fetal bovine serum.

Moreover, cells from other tumor types were obtained from a panel of 60 human tumor cell lines, representing nine tumor tissue types, used by the National Cancer Institute (NCI) Anticancer Drug Screening Program. For pancreas tumor the cell lines selected were: IMIMPC2, MiaPaCa, Aspcl, A6L, SKPC-1 and Panc-1; for colon cancer: CACO. SW480 and SW1222; for bladder cancer: RT112, MGHU4, 639V, 253J, MGHu3 and SW1170; for glioma and glioblastoma: U87MG, U251 and T98G; for breast cancer: MDA-231, MCF7 and T47D; for prostate cancer: LNCaP, PC3 and DU145; for lung cancer: H1299 and NCIH460; and for ovarian cancer: NCI H23 and SK-OV-3.

All cells were cultured in Dulbecco's modified Eagle's medium (Life Technologies, Rockville, MD, USA) supplemented with 10% fetal bovine serum (Nova-Tech Inc., Grand Island, NY, USA).

### Generation of PEI complexed pIC (BO-110).

The synthetic analog of dsRNA, pIC, was purchased from InvivoGen (San Diego, CA). The reactive jetPEI^{™}, jetPEI-Fluor^{™} and invivo-jetPEI^{™} were adquired from Polyplus-transfection (Ikirch, Francia) These products, which contains a lineal derivative of poliethemine, were used to complex pIC at a N/P ratio (nitrogen residues of JetPEI per RNA phosphate) of 1 to 5 *in vitro* and *in vivo*, according to the manufacturer's protocol.

Unless otherwise indicated, the concentrations of pIC used in cultured cells were of 1 µg/ml and 1-2 ng/kg in mice.

### Drug treatments and cell death assays.

Bortezomib (Velcade, formerly PS-341) was obtained from Millenium Pharmaceuticals Inc (Cambridge, MA); Adriamycin (doxorubicin) from Sigma Chemical (St.Louis, MO), and etoposide from Bristol-Myers Squibb (New York, NY). The antioxidant Tiron and Vit-E were purchased from Sigma (St. Louis, MO), and the pan-caspase inhibitor ZVAD from R&D System (Minneapolis, MN). 3-methyladenine (3-MA) was obtained from Sigma Chemical (St.Louis, MO). Chloroquine was obtained from Sigma Chemical (St Louis, MO, USA).

Cell viability assays in response to drug treatments were done after seeding melanocytes and melanoma cells at least 12 hours before drug treatment. The percentage of cell death at the indicated times and treatment concentrations was estimated by standard trypan blue exclusion assays as previously described (Wolter et al., 2007; Fernández et al., 2005).

Cell proliferation assays in response to drug treatments were performed after seeding tumor cells at least 12 hours before drug treatment. The growth of cells at the indicated times and treatment concentrations was estimated by crystal violet staining assay.

### Protein immunoblotting.

To determine changes in protein levels, 2x10⁶ cells were treated as indicated and harvested at different times after treatment. Total cell lysates were subjected to electrophoresis in 10, 12 or 4-15% gradient SDS gels under reducing conditions, and subsequently transferred to Immobilon-P membranes (Millipore, Bedford, MA, USA). Protein bands were detected by the ECL system (GE Healthcare, Buckighamshire, UK).

Primary antibodies included: casp-9 and-3 from Novus Biological (Littleton, CO, USA); casp-8 (Ab-3) from Oncogene Research Products (San Diego, CA, USA), casp-7 from Cell Signaling Technology (Beverly, MA, USA); Bcl-xL from BD Transduction Laboratories (Franklin Lakes, NJ, USA); Blc-2 from Dako Diagnostics (Glostrup, Denmark); NOXA from Calbiochem (San Diego, CA, USA); MDAp53 from Novocastra Laboratories (Newcastle, UK); and tubulin (clone AC-74) from Sigma Chemical (St Louis, MO, USA). The MDA-5 antibody has been described before.

Secondary antibodies were either anti-mouse or anti-rabbit from GE Healthcare. Image J was used to quantify changes in proteins levels induced by the different treatments, considering untreated controls as reference for basal expression.

### RNA interference.

The shRNA lentiviral vector used to downregulate NOXA has been previously reported (Fernández et al., 2005). The plko lentiviral vector used to downregulate MDA-5 (target sequence, SEQ ID NO: 1) were purchased from OpenBiosystems (Huntsville, AL). Scrambled oligonucleotides were also designed to generate control shRNA. Viruses were generated from 293FT cells as described and used under conditions that render > 80% infection efficacy (Denoyelle et al., 2006). The downregulation of MDA-5 was confirmed by immunoblotting and RT-PCR (forward primer of SEQ ID NO: 2 and reverse primer of SEQ ID NO: 3). When indicated, treatment with pIC or BO-110 was initiated 3 days after infection with the corresponding shRNA-expressing viruses.

### Expression profiling Microarrays.

Total RNA was isolated from at least two independent experiments and was purified with the RNeasy Kit (Quiagen). Treated samples with BO-110 were labeled with 2.5 mg Cy5- UTP and used as reference in the hybridization reactions with 2.5 g of RNA labeled with Cy3-dUTP resulting from incubation with PIC or PEI. Marked RNA was hybridized two colors oligos Full human genome Microarray (4x44K) from Agilent (Santa Clara, CA, USA) following the manufacturer instructions. After washing, the slides were scanned using a Scanarray 10 5000 XL (GSI Lumonics Kanata, Ontario, Canada) and images were analyzed with GenePix 4.0 program (Axon Instruments Inc., Union City, CA), as described previously (Alonso et al., 2007). Intensity measures of fluorescence were subjected to automatic background subtraction. Relationships Cy3: Cy5 were normalized to the value of the median ratio of all points. After normalization, points with intensities for both channels (sum of medians) lower than the local background were discarded. The relations of the remaining points were subjected to logarithmic transformation (base 2), and duplicate points arrays were adjusted to the median. The grouping of pairs not weighted (UPGMA: unweighted pair-group) of genes expressed in a differential between control and test samples was conducted with the Gene Expression Pattern Analysis Suite (GEPAS).

### Treatment response in vivo.

Female C57BL/6 mice were purchased from NIH (Bethesda. MA). Female SCID Beige mice, which have impaired NK, T and B cell lymphocyte function, were from Charles Rivers (Wilmington, MA). All animals were 6-12 weeks of age at the onset of experiments. Animal care was provided in accordance with institutional procedures at the University of Michigan Cancer Center.

Engraftments in the skin were generated by intracutaneous injection of 10⁵ B16 melanoma cells. 2 ng/kg µg pIC alone or complexed with invivoJetPEI were administered by peritumoral injections on days 7, 11, 15 and 21 post tumor implantation. Additional treatment groups included JetPEI as single agent and placebo controls. Tumor volume was estimated by caliper measuremens and calculated as *V*= *L x W²l2*, where *L* and *W* stand for tumor length and width, respectively.

Surrogate models of lung metastasis were generated by i.v injection of 4x10⁵ B16-eGFP or 5x10⁵ SK-Mel-103-eGFP melanoma cells. Treatment was performed on day 3, 6, and 9 by i.v. injection of 1ng/kg of pIC alone or complexed with *invivoJetPEI*. Lungs were harvested 14 days after challenge and external metastases were counted manually and scored by number and size. Alternatively, an Illumatool TLS LT-9500 fluorescence light system (Lightools Research, Encinitas, CA, USA) and the emitted fluorescence from tumor cells was captured with a Hamamatsu Orca 100 CCD camera. Metastatic involvement was monitored independently by analysis of hematoxylin-eosin staining of paraffin sections. Experiments were done in groups of five mice and repeated two to four times: Mice were euthanized when control populations showed signs of discomfort or respiratory defects.

Autochthonous melanomas were generated crossing Tyr:: N-RasQ61K mice with Ink4a/Arf knockout mice in a C57BL/6 background (Ackermann et al., 2005). For the induction of melanoma in the skin, mice were painted once, at the age of 8-10 weeks with 220 mg of 7,12-dimetilben[a]anthracene (DMBA). After the development of early melanocytic neoplasms (lesions of at least 1 mm in diameter), mice were treated two times per week with intraperitoneal injections of 1 ng/kg as a single agent or pIC complexed with in vivoJetPEI. Melanoma and moles (nevi) were counted and its size was measured in two diameters using a caliper, expressed as average size tumor in mm3.

The size of tumors was also evaluated by PET-CT (Positron Emission Tomography-Computed Tomography). The exploration and acquisition of PET-CT images was performed with the PET-CT system for small animals View Explore General Electrics (Fairfield, CT, USA). 15 MBq of 18F-FDG (2-fluoro-2-deoxy-D-glucose) were injected for imaging and adquisition of PET images and reconstructed using the algorithm 3DOSEM. The CT images were acquired in 16 shots with energy of 35 KeV and 200 uA, and the images were reconstructed using the FDK algorithm. Melanomas, metastases, and other organs were monitored independently by analysis of paraffin sections stained with hematoxylin-eosin.

### Transmission electron microscopy.

For transmission electron microscopy (TEM), the indicated cell populations were rinsed with 0.1 Sorensen's buffer, pH 7.5 and fixed in 2.5% glutaraldehyde for 1.5 h, and subsequently dehydrated and embedded in Spurr's resin. Then, the block was sectioned at 60-100 nm ultra thin sections and picked up on copper grids. For routine analysis ultrathin sections were stained with 2% uranyl acetate and lead citrate. Electron micrographs were acquired with a Philips CM-100 transmission electron microscope (FEI, Hillsbrough, OR) and a Kodak 1.6 Megaplus digital camera.

### Confocal and fluorescence microscopy: Quantification of the GFP-LC3 punctuated dots.

An eGFP-LC3 fusion cloned into the pCNA expression vector was a gift from Gabriel Núñez (University of Michigan Cancer Center). eGFP-LC3 and the fragments eGFP-Rab7wt, eGFP-Rab7 T22N, eGFP-Rab5wt, eGFP-Cherry-LC3 and Cherry-LC3 were cloned into the pLVO-puro lentiviral vector for stable gene transfer. Melanoma derived cells (ie. SK-Mel-103) were infected with pLVO-eGFP-LC3 and selected with puromycin. GFP-LC3-associated fluorescence emission was imaged using a Leica AF6000 fluorescence microscope and images were analyzed by LAS AF V1.9 (Leica, Solms, Germany). For confocal real time microscopy, we used a Leica TCS-SP2-AOBS-UV ultra-espectral microscope coupled to a CO₂ and temperature-controlled incubation chamber. Images were analyzed by LCS (Leica, Solms, Germany). For colocalization experiments the Lysotracker^{™} Red or Blue (Invitrogen, Carlsbrad, CA) at a concentration of 50nM or 200nM and Hoescht 33342 (Invitrogen, Carlsbrad, CA) were added 10 minutes before imaging at a concentration of 5ug/ml. Co-localization images were analyzed with LAS AF V1.9 (Leica, Solms, Germany).

### Cytokine expression.

Human interferon alpha was measured in culture supernatants by enzyme-linked immunoabsorbent assay (ELISA). The human IFN-α ELISA Kit and recombinant hIFN-α were purchased from PBL Interferon Source (Piscataway, NY) and used according to the manufacturer's protocol. IFN-α expression level was measured from bone-marrow-derived macrophages (BMDMs) and B16 melanoma cells by real-time PCR. BMDMs were prepared, plated and treated as previously described (Celada et al., 1984). Real-time quantitative PCR analysis of IFIT-1 RNA transcripts was performed using TaqMan primer and probes obtained from Applied Biosystems on an Applied Biosystems 7700 sequence detector after normalization with β-actin.

### Statistical analyses.

Viability data are expressed as means +/- s.e.m, and statistical analysis of the differences was determined by the two-tailed Student's t-test. *P* < 0.05 was considered significant. For statistical evaluation of tumor growth and metastasis *in vivo*, the generalized Mann-Whitney Wilcoxon test was used to compare the values of continuous variables between two groups. *P* values of <0.05 were considered significant.

### Example 1. Identification of autophagy inducers in melanoma cells by a discriminative analysis based on LC3 fluorescence. Confirmation of autophagic cell death induced by BO-110 through ultrastructural analysis.

As discussed previously, a hallmark of autophagy (for both induction of cell death and survival), is the relocation of the autophagy protein gene 8 (ATG8) / LC3 from the cytosol to the newly generated autophagosomes. Based on this observation, changes in the cellular distribution of a GFP-LC3 fusion protein (i.e. from a diffuse pattern to a focal staining) are used as a marker for early stages of autophagy. The presence of autophagosomes can also be confirmed by electron microscopy or light microscopy.

### 1.1. Fluorescence analysis based on LC3.

To address the role of autophagy in the response of melanoma to drugs, a discriminative analysis based in GFP-LC3 was used to screen commercially available chemotherapeutic drugs and immunomodulators. Melanoma cells were stably transfected with lentiviral vectors expressing derivatives of the autofagosomal LC3 marker with GFP, such as pLVO-eGFP-LC3.

The human cell line SK-Mel-103 was selected as the model system for the initial screening based on its highly metastatic and chemoresistant phenotype (Soengas et al., 2001). Subsequent validation studies were performed on a panel of human cell lines of diverse genetic background (see below).

A variety of anticancer drugs were found to induce focal GFP-LC3 fluorescence emission without significantly affecting cell viability. However, among death inducers, a complex of PEI and the dsRNA mimetic polyinosine-polycytidylic acid (BO-110) was found to be particularly efficient at engaging GFP-LC3 foci. About 50% of cells showed noticeable punctate GFP-LC3 staining within 4-6 h of incubation in low doses (0.5-1 µg/ml) of BO-110 (see representative micrographs and quantifications in **Fig. 1A**, in **Fig. 1B**). In fact, kinetic analyses indicated a faster generation of GFP-LC3 foci by BO-110 than by rapamycin (**Fig. 1B**), a classical positive control for autophagy induction (Klionsky et al., 2008).

Interestingly, at late time points, BO-110 treatment was able to induce cell death, even in melanoma cell lines that are intrinsically resistant to standard DNA damaging agents such as doxorubicin or etoposide, like the case of SK-Mel-103

The analysis of the endogenous LC3 showed changes in the electrophoretic mobility (**Fig. 1C**), corresponding to the characteristic lipidation of this protein during autophagosome formation and the requirement of ATG5 for the generation of GFL-LC3 foci formation.

The authors of the invention were not aware of any previous report that would have linked the pIC to autophagy in cancer cells. Therefore, the following tests were focused on this compound, since it could reveal new elements to enhance the understanding of the potential intracellular sensor of dsRNA to induce autophagy and tumor cells death.

### 1.2. Ultrastructural analysis of the BO-110 effect on cells.

The GFP-LC3 focal staining in cells treated with BO-110 described in the previous paragraph is consistent with the formation of autophagosomes. However, to rule out possible unspecific aggregations of ectopically expressed GFP-LC3 (Klionsky et al., 2008), the response was analyzed independently by electron microscopy (**Fig. 1D**).

Early responses (5 h) to BO-110 involved a marked accumulation membrane-bound electron dense structures sequestering cellular debris (**Fig. 1D**), a distinctive feature of autophagosomes. The size and number of these structures were visible as intracellular granules even by optical microscopy (**Fig. 1E**).

At later times points, the treatment with BO-110 induced the cellular collapse (**Fig. 1F**, middle panel), which was found by electro microscopy to be associated to the formation of large phagocytic vacuoles, of more than 500 nm diameter (**Fig.1F**, right panel).

**Fig. 2** shows a summary of the evolution in time of autophagy induction by selected fluorescence micrographs taken at different times, which can be seen in micrographs of EGFP redistribution over time: from a diffuse staining to focal aggregates, indicating the formation of autophagosomes, a process that culminates in a generalized cell collapse. Control cells showed a diffuse staining throughout the testing period, indicative of basal levels of accumulation of LC3.

It is interesting that the integrity of plasmatic and nuclear membranes remained, and that cells treated with BO-110 showed the characteristic chromatin condensation of apoptosis programs. The induction of autophagy was dependent on BO-110, as PEI (Control) had minimal impact on the number or size of the autophagosomes (**Fig. 1D**-**F**). Taken together, these findings support a cytotoxic effect of BO-110 that involves the formation of autophagosomes, followed by cell death with apoptotic-like features.

### Example 2. Sensitivity analysis of different human and murine melanoma cell lines with pIC conjugated to cationic molecules and selectivity against melanocytes.

To determine whether the activity of pro-autophagic BO-110 found in the initial study conducted with SK-Mel-103 cells was a reflection of a more broadly anti-melanoma activity, an additional set of cell lines were tested.
Melanoma cells were selected to correlate with frequent melanoma associated events, such as mutations in *BRAF* or *NRAS*, deletion of *INK4a*/*ARF* or *PTEN* loci, or upregulation of various anti-apoptotic Bcl-2 family members, which are known to contribute to the progression and chemioresistance of melanoma.

P53 mutations are rare in melanoma (Soengas and Lowe, 2003). However, since p53 may play a key role in the activation of apoptotic and autophagic programs, we also performed tests on SK-Mel-28 cell line that expresses a mutant p53, to determine whether this tumor suppressor is strictly required for the activity antimelanoma of BO-110. In addition, a murine metastatic melanoma cell line B16 was also included in the analysis as an example of model widely used in immunotherapy of melanoma (Wenzel et al., 2008), to assess differences in treatment response supposedly associated with differences between species. In parallel, we also analyzed melanocytes isolated from human foreskin.

Shown in Table 1 is the genetic background of the human metastatic melanoma cell lines:

**Table 1. Genetic background of the human metastatic melanoma cell lines.**

| Cell line | p53 | Induc. p53 | p14 (mRNA) | p16 (mRNA) | BRAF (V599) | NRAS (exón 3) | PTEN (prot) | Apaf-1 (prot) | Casp8 (prot) | Bcl-2 (prot) | Bcl-xL (prot) | Mcl1 (prot) | DOX |
|---|---|---|---|---|---|---|---|---|---|---|---|---|---|
| **SK-Mel-19** | wt | + | + | +** | mut. | Tipo silv. | - | ++ | ++ | +++ | ++ | ND | ++ |
| **SK-Mel-28** | L145R | -* | ND | +** | mut. | Tipo silv. | + | -/+ | ++ | ++ | ++ | ++ | - |
| **SK-Mel-103** | wt^{R} | + | + | + | wt | Q61R | + | - | + | ++ | +++ | +++ | - |
| **SK-Mel-147** | wt^{R} | + | - | +** | wt | Q61R | + | - | + | ++ | +++ | +++ | - |

p53 mutational status was determined by direct sequencing of exons 2-10 by RT-PCR. Samples with polymorphism P72R are indicated as R. The inducibility of p53 was determined by immunoblotting of extracts treated with doxorubicin (0.5 mg/ml, 12h). Lines with high endogenous levels of p53 are indicated with an asterisk. PTEN, Apaf 1, Casp-8, Bcl-2, Bcl-xL and Mcl-1 levels were determined by immunoblotting and normalized to control melanocytes. BRAF and NRAS mutational status was determined by direct sequencing of PCR-amplified genomic fragments of exons 15 and 3 respectively. Responses to doxorubicin (DOX; 0.5 µg/ml, 30h) are categorized into ++, +, -/+, -, for percentages of cell death of 100-70, 70-50, 50-30 and <30%, respectively. Responses to BO-110 (1ug/ml, 30h) are categorized into +++, ++, +, for percentages of cell death of 100-90, 90-60 and 60-40%, respectively. Lines with high endogenous levels of p53 are indicated with an asterisk..

With these lines, tests were conducted similar to those described in Example 1 for SK-Mel-103 cell line, to verify the sensitivity of each of these cells to pIC and PEI as independent agents or used in combination. The results are summarized in **Fig. 3**.

The five melanoma cell lines tested (SK-Mel-19, -28, -103, -147, and B16) were killed with similar kinetics and sensitivity after treatment with BO-110 (**Fig. 3A**). It is significant to point out that in all melanoma lines in which the test was conducted; the early activation of autophagy by BO-110 was invariably followed by cell death. Electron microscopy showed clear autophagosomes, also in the case of the p53 mutant line, SK-Mel-28 (**Fig. 3B**) As shown in **Figure 3A** and the dose-response curves depicted in **Fig. 4B** (which shows representative data of the lines corresponding to four independent isolates), it is important to note that, under conditions that caused the death of 70% of SK-Mel-103 melanoma cells 24 hours after treatment, normal melanocytes remained viable and showed no markers of autophagy.

Moreover, as shown in **Fig. 4A**, no significant changes in morphology, granulation or cytosolic GFP-LC3 distribution in melanocytes over a wide range of concentrations BO-110 were observed. **Fig. 4C** is also indicative that normal human skin fibroblasts were also more resistant to BO-110 than melanoma cells

It is interesting to find that PEI is critical in its selectivity for tumor cells. Thus, the antimelanoma activity of pIC was reduced by 70-80% when PEI was not included in the treatment (**Fig. 3A**). Without PEI, the naked pIC was almost as ineffective in melanoma cells and in melanocytes, and showed minimal activity as inducer of autophagy (**Fig. 3B**).

PEI is a classic vehicle in gene therapy for its ability to promote uptake of DNA and RNA molecules by endocytosis (reviewed in Payne, 2007). The multilaminar structures found in melanoma cells treated with BO-110 (see pictures below of **Fig 2B**), are in fact consistent with multiple fusion events involving the arrival of endosome-lysosome hybrids (amphisomes) to autophagosomes (Maiuri et al., 2007).

### Example 3. Qualitatively different activation of MDA-5 by pIC in the absence and presence of PEI.

In addition to favouring endocytosis of DNA or RNA molecules, PEI can promote endosomal swelling and allowing an efficient delivery of genetic material to the cytosol (revised in Payne, 2007). Therefore, PEI could favour the access of pIC to intracytosolic sensors. The Melanoma Differentiation-Associated gene-5 (MDA-5) is one of these sensors (Akira, 2006), and therefore the authors tested whether this protein was the driver of BO-110-mediated killing of melanoma cells.

Activation of MDA-5 was analyzed by monitoring the proteolytic cleavage that separates its helicase and caspase activation recruitment domains (CARD) during cell death, as described (Kovasovics et al. 2002; Barral et al., 2007).

This analysis was carried out by immunoblotting extracts from SK-Mel-28 and SK-Mel-147 cells treated with PEI, pIC or BO-110, after subjecting these extracts to electrophoresis. As a positive control for efficient induction of cell death bortozomib was used. The results are shown in **Fig. 5****.**

Interestingly, protein immunoblotting revealed a strong and sustained ability of the PEI-pIC complex, BO-110 to induce the processing of MDA-5 (**Fig. 3A**). Naked pIC could induce this processing, albeit to significantly lower levels, and not in all cells tested nor in a sustained manner (none of the lanes corresponding to SK-Mel-28 cells show the band of 30 kD, characteristic of the existence of proteolytic cleavage, which should appear at the height of the arrow in panels A and B of **Fig. 5**) or sustained (see **Figure 5A****,** where the intensity of the band of 30 kD decreases with the time of pIC treatment in SK-Mel-147 cells).

To define the contribution of MDA-5 to the cytotoxic activity of BO-110, short hairpin RNAs (shRNA) complementary to MDA-5 were transduced into melanoma cells via lentiviral vectors for stable knockdown of MDA-5 (see protein immunoblots in **Fig. 3B**). MDA-5 shRNA significantly reduced BO-110 driven melanoma cell death with no detectable unspecific effects on control cells (**Fig 3C**, *p*<0.05).

Importantly, the induction and processing of MDA-5 was not simply a consequence of the activation of the death machinery in melanoma cells. Treatment with bortezomib, a proteasome inhibitor able to activate both the intrinsic and death receptor apoptotic pathways in melanoma had no effect on MDA-5 levels or processing (**Fig. 5A**). These results illustrate main mechanistic differences in the execution of death programs by BO-110 and other pro-apoptotic inducers.

### Example 4. Pharmacological inhibitors of autophagy compromise the cytotoxic activity of BO-110.

Next, the assays focused on the mechanisms involved in the execution of BO-110-induced cell death. 3-methyladenine (3-MA) and chloroquine are frequently used for independent validation of autophagy mechanisms by their ability to interfere with autophagosome formation or autolysosomal activity, respectively (Maiuri et al., 2007; Klionsky et al., 2008).

To check whether this interference occurred in melanoma cells treated with BO-110, SK-Mel-103 melanoma cells were subjected to treatment with 3-methyladenine or chloroquine 12 hours after treatment with BO-110 or with buffer control (vehicle). The results are shown in **Fig. 6****.**

As shown by fluorescence microscopy (**Fig**. **6A** and **Fig. 6B**), 3-MA blocked GFP-LC3 foci formation by BO-110. In the presence of chloroquine, autophagosomes accumulated, but interestingly, this induction was not productive as a death inducer (**Fig**. **6C****,** It is observed that the percentage of dead cells observed in presence of chloroquine is lower than that observed with pepstatin A, E64d, or a combination of both). Therefore, these results support a scenario where the cytotoxic activity BO-110 is not a passive byproduct of autophagosome formation: the lytic activity of the lysosome is an essential mediator ofBO-110-killing of melanoma cells.

### Example 5. BO-110 drives autophagosome/lysosome fusion to subsequently engage death programs.

If the autolysosome is a key driver of BO-110, blockage of lysosomal hydrolases should protect melanoma cells during BO-110 treatment. It is not feasible to block all lysosmal-dependent activity as multiple enzymes with overlapping targets can localize in this organelle (Fehrenbacher and Jaattella, 2005). Still, useful information on lysosomal activity can be provided by, the broad spectrum protease inhibitors E64d and pepstatin A are as these compounds efficiently block various cathepsins (B, D, and L) in autolysosomes (Klionsky et al., 2008). Therefore, a trial was conducted to compare the effect of chloroquine, pepstatin A or E64d on cell death 20 hours after treatment with buffer control or BO-110. Results are shown in **Fig. 6C****,** which has been mentioned previously. Notably, pestatin A and E64d reduced by 50% the extent of cell death by BO-110.

To confirm that the vesicles corresponded to the previously of large multivesicular structures identified that involved large endosomes, which in turn recruited multiple autophagosomes (to generate hybrid structures known as amphisomes) and these vesicles weren't a result from halted autophagosomes in which lysosomes are either not recruited or dysfunctional or autofagosomes were a result of accumulation of inadequate degraded material, melanoma cells were transfected with fusions of GFP and Cherry-LC3. Cherry-GFP-LC3 signals leads to red and green fluorescence autophagosomes, due to the two fluorescence proteins (Cherry and GFP), but they loose the GFP signal (green) in the acidic environment of autolysosomes.

Using this strategy revealed that, in fact, BO-110, similar to rapamycin, induced the formation of autolisosomas in melanoma cells, as indicated by the presence of red-only LC3 foci, in **Fig. 6C****.** The chloroquine, consistent with previous trials, blocked melanoma cell death triggered by BO-110 (**Fig. 6D**), without affecting the endosomal uptake of this dsRNA mimic (as determined by colocalization of FluoRed-labeled BO-110 with GFP-fused early endosomal protein Rab5 (**Fig. 6E**). Similar inhibitory effects were observed using the broad spectrum protease inhibitors E64d and pepstatin A and the vacuolar ATPase blocker bafilomycin (**Fig. 6D**), supporting a new mode of action of BO-110 dependent on lysosomes.

To independently monitor lysosomal activity during BO-110 treatment, cells were tested for the ability to process DQ-BSA (a derivative of BSA whose green fluorescence is quenched unless cleaved by proteolytic enzymes). As shown in **Fig. 6F****,** DQ-BSA was efficiently cleaved in the presence of BO-110. Note that DQ-BSA emission was detected at the lysosomes, as indicated by colocalization with Lysotracker red, a dye whose cell permeability is pH dependent and emits red fluorescence when incorporated into functional lysosomes acids). The result contrasts with the minimum of fluorescence emission due to DQ-BSA observed in the SK-Mel-103 cells treated with BO-110 when lysosomal activity was blocked with chloroquine (**Fig. 6F** and **Fig 6G**).

To further characterize the ability of BO-110 to cause the initiation and complete development of autophagy process, the autophagosomes and lysosomes fusion was visualized by confocal microscopy. For this purpose, SK-Mel-103 cells stably expressing GFP-LC3 were treated with BO-110 or corresponding buffer as a control, and incubated in the presence of Lysotracker-Red. The dual emission analysis of green and red fluorescence (for GFP-LC3 fusion and Lysotracker, respectively), based on individual cells and the cell population showed a clear colocalization of autophagosomes and lysosomes (see representative fluorescence photomicrographs in **Fig. 6H** and **6I** and the corresponding quantifications in **Fig 6****J**). It is important that this colocalization was an early event in the responses triggered by BO-110 (already detectable in the 4 - 8 hours after treatment), and preceded an organized cell collapse.

Having determined that autophagosomes fuse to active lysosomes in response to BO-110, we assessed whether these organelles interacted with or were recruited to endosomes. First, endosomal dynamics were assessed in melanoma cells expressingGFP fused to the late endosomal marker Rab7 (Luzio et al., 2007. Basal endosome generation and resolution (i.e., progressive reduction in size) was detected in untreated melanoma cells (left panel from **Fig. 7A**). However, BO-110 treatment markedly enhanced endosomal activity, inducing a sustained and multiwave generation of endosomes (Middle and right panel from **Fig. 7A**). These endosomes were found to be filled with lysosomes, as determined by dual imaging of GFP-Rab7 and Lysotracker red (**Fig**. **7B**). Moreover, time-lapse microscopy revealed fast kinetics of multiple recruitments of lysosomes to GFP-Rab7-decorated endosomes as also shown in the sequential series of fusion events in **Fig. 7C****.** Importantly, as shown in **Fig. 7B** (right panels), endosome-lysosome fusion was significantly inhibited if cells overexpressed Rab7-T22N, a known dominant- negative mutant of this protein. In total, these results uncovered a dynamic mobilization of endo/lysosomal compartments in tumor cells treated with BO-110.

### Example 6. BO-110 links autophagy to apoptotic caspases.

Lysosomal proteases can impact on death programs at multiple levels (Maiuri et al., 2007; Hoyer-Hansen and Jaatella, 2008). In the case of the mitochondria, they can dysregulate the production of reactive oxygen species (ROS) and/or engage classical apoptotic caspases (the regulatory casp-9 and the effector casp-3 and -7). Extrinsic pathways dependent on the casp-8 can also respond to lysosomal activation (Fehrenbacher and Jaattella, 2005. To address the implication of ROS in the mode of action of BO-110, treatment was performed in the presence of vitamin E, Trolox or Tiron, scavengers with a different antioxidant activity and the pan-caspase inhibitor z-VAD-fmk. The results when analyzing the results on cell death in each of these cases are shown in **Fig. 8A****,** which data obtained with vitamin E are shown as a representative case of the results obtained with chemical antioxidants mentioned at the doses of these reagents that block apoptosis in melanoma controlled by ROS (Fernández, 2006). As shown in the figure, the presence of these antioxidant agents, no significant effects were observed on cell death by BO-110. Instead, the pancaspase inhibitor z-VAD-fmk inhibited BO-110 killing by 70%. Altogether these results support a caspase-dependent mechanism activated downstream of an autophagy program.

Caspase processing was in fact efficiently promoted by BO-110 as determined by immunoblotting of cell extracts collected after different times after being subjected to no treatment (NT) except the buffer control without PEI or treatment with PEI, pIC, the complex BO-110 or the known inducer of caspases cleavage, bortezomib (**Fig**. **8B** and **Fig. 8C**). **Figure 8B** compares the ability of PEI, pIC and BO-110 to induce apoptotic processing of caspases 8 and 9 in different lines of metastatic melanoma: effective activation of caspases 9 and 8 was clearly evident 20h after treatment with BO-110 in all human melanoma cell lines tested; similar to what was observed with bortezomib. Moreover, the kinetics and extent of caspase processing by BO-110 were highly consistent (**Fig. 5B**), independently of the mutational status of BRAF (e.g. SK-Mel-19), NRAS (SK-Mel-103, -147), or p53 (SK-Mel-28).

**Fig. 8C** shows the results of a similar test conducted with the SK-Mel-103 line, which undertook a more complete analysis including, in addition to the apoptotic caspases 9 and 8, the effector caspases 3 and 7. The same test was carried out with the SK-Mel-147 line, which gave similar results. The efficient processing of the casp-9, -3 and -7 in SK-Mel-103 and -147 was particularly relevant. These lines have low levels of Apaf-1 and are very inefficient at engaging the casp-9/Apaf-1 apoptosome (Fernández et al., 2005; Soengas et al., 2006) in response to classical anticancer agents such as doxorubicin, etoposide or cisplatin (see graphic in **Fig. 1C**). Therefore, these results show a superior ability of BO-110 to activate apoptotic programs and bypass inherent mechanisms of resistance to standard chemotherapeutic drugs.

### Example 7. Activation of cell death by BO-110 in the absence of compensatory effects on anti-apoptotic Bcl-2 family members.

To analyze in more detail the mode of action of BO-110, and to identify events that may be uniquely activated by this agent, drug response was compared to the effects of bortezomib. This agent was selected because it is also a potent activator of the apoptotic machinery in melanoma cells (Wolter et al., 2007; Fernández et al., 2006)

However, we expected bortezomib and BO-110 to be mechanistically distinct. Bortezomib targets the proteasome and not the lysosome(Qin et al., 2005). Moreover, as shown in **Fig. 5A****,** bortezomib kills melanoma cells without inducing or processing MDA-5. Bortezomib is also interesting as it can promote a massive accumulation of the pro-apoptotic NOXA, but also induce a rapid and drastic upregulation of its antiapoptotic antagonist factor MCL-1 (Fernandez et al., 2005), a member of the Bcl-2 family. Importantly, MCL-1 acts as an internal compensatory mechanism to proteasome inhibition and blocks the antitumorigenic effect of Bortezomib *in vitro* and *in vivo* (Wolter et al., 2007; Qin et al., 2006).

To assess similarities and differences between bortezomib and pIC (naked or complexed with PEI), melanoma cells were incubated with each of these compounds and extracts were collected at different time points after treatment to assess the levels of NOXA, MCL-1, and other Bcl-2 family members (Bcl-xL or Bcl-2). Results are shown in **Fig. 9****.**

As shown in panels **A** and **B** from **Fig. 9****,** naked pIC failed to induce NOXA consistently or in a sustained manner in SK-Mel-28 or SK-Mel-147 melanoma cells (cells that express p53 L145R mutation or p53wt, respectively). On the other hand, BO-110 induced NOXA by 35, 10 and 5-fold over basal levels in SK-Mel-28, SK-Mel-147 and SK-Mel-103, respectively (see immunoblots in panels **A** and **C** from **Fig. 9****,** and representative quantifications in **Fig. 9B** from the resultsobtained in SK-Mel-28 cells), again emphasizing the differential activity of naked and PEI-complexed pIC.

With respect to inhibitory regulators of NOXA, MCL-1 levels were minimally induced by BO-110 (**Fig**. **9A** and first graph from **Fig. 9B**). This is in contrast to bortezomib, which activates NOXA potently, but induces a simultaneous accumulation of MCL-1, as previously described (Fernández et al., 2005). Other anti-apoptotic Bcl-2 family members such Bcl-2 and Bcl-xL were also not affected by BO-110 (see immunoblots for SK-Mel-103 in **Fig. 9C**).

In the absence of compensatory mechanisms, the relatively lower levels of BO-110 induced NOXA could be sufficient to promote cell death. To test this hypothesis, melanoma cells were transduced with a shRNA previously demonstrated to inhibit NOXA mRNA and protein specifically (Fernández et al., 2005), and using as a control cells infected with a lentiviral vector expressin an inactive shRNA control. As shown in **Fig. 9D****,** a 50% reduction in NOXA protein expression by shRNA inhibited NOXA upregulation by BO-110 also nearly by 50%, and inhibited BO-110toxicity (**Fig. 9E**).

Next, we used shRNAs against MDA-5 to define the requirement of this protein for the regulation of NOXA by BO-110. and an essay was performed as previously but quantifying NOXA levels. Results are shown in **Fig.9F** graph. Interestingly, MDA-5 shRNA inhibited NOXA protein levels by 70% (**Fig**.**9F**), without secondary effects on other Bcl-2 family members.

Together these results uncovered a new point of action of MDA-5 in the apoptotic machinery driven by the induction of NOXA.

### Example 8. Differential efficacy of naked pIC and BO-110 in immunocompetent mice.

Next, anti-melanoma activity of pIC and BO-110 was assessed *in vivo.* In melanoma models, naked pIC has to be administered either at high doses or in combination with other agents (e.g. protein synthesis inhibitors) for an effective activation of innate immunity programs. The data obtained in the previous examples suggested that pIC will be significantly more potent in the presence of PEI.
Treatment response was first analyzed in an immunocompetent background. B16 mouse melanoma cells, either untransduced or transduced with GFP (to ease detection by fluorescence imaging), was implanted in syngeneic normal mice. Two strategies were used: injection of tumor cells (i) subcutaneously (s.c.) or (ii) intravenously, to assess tumor progression at localized sites or as distant metastases, respectively. Mice were treated with PEI, pIC or BO-110 or 100ul glucose 5% (NT group)

**Fig. 10A** summarizes the experimental strategy for the s.c xenotransplants with B16 generation and the dosing and treatment schedule. At the treatment times peritumoral injections of 2 ng/kg of naked pIC or complexed with PEI were injected.

Notably, BO-110 was found to be superior to pIC in all cases studied. Thus, mice with subcutaneously growing B16 melanomas which received vehicle, PEI or pIC alone had to be sacrificed within 15-25 days after implantation, due to excessive tumor growth (**Fig. 10A**). Under the same conditions, subcutaneous melanomas in the BO-110 treatment group were either undetectable or significantly smaller (**Fig. 10A**).

**Fig. 10B** summarizes the experimental strategy to implant intravenously B16-eGFP melanoma cells and the subsequent treatment with pIC, PEI, BO-110 or glucose 5% (NT group). In this figure are also shown fluorescence images from the sacrificed animal lungs **(****Fig. 10B** and the lung metastases quantification (**Fig. 10C**). In this experiment, BO-110 was 5-fold more potent than naked pIC also in surrogate models of melanoma lung metastasis, as determined by fluorescence imaging.

### Example 9. IFN does not recapitulate the death-inducing features of BO-110.

pIC is a classical inducer of IFN-driven cellular immunity (Wenzel et al., 2008). The data exposed in the previous examples suggested, however that pIC, when complexed to PEI, could also act in a cell autonomous manner, which may be distinct from IFN-mediated responses in "professional" immune cells. To assess this possibility, B16 melanoma cells and macrophages were tested for their ability to secrete and respond to IFN-α. RT-PCR indicated that both cell types activated classical IFN-α targets such as IFIT-1 (IFN-induced protein with tetratricopeptide repeats) after treatment with BO-110 (**Fig. 11A**). PEI was dispensable for pIC-mediated induction of IFN targets in macrophages (**Fig. 11A**). This is expected, as these cells can efficiently sense viral dsRNA. Melanoma cells, however, were unable to induce IFIT-1 just with naked pIC (**Fig. 11A**).

For a direct assessment of IFN-α production by melanoma cells, an Elispot assay was performed, using recombinant human IFN-α as a reference control. IFN-α levels secreted by melanoma cells after BO-110 treatment were lower than 10 pg/ml. To determine whether IFN-α can substitute for BO-110 (i.e. whether IFN-α secretion is the main inducer of melanoma cell death), increasing amounts of this cytokine were added to melanoma cells. Interestingly, high doses of IFN-α (10 times over the levels secreted after treatment BO-110) were unable to affect melanoma cell viability (**Fig 11B**).

It is interesting to note also that the microarrays tests showed that response to pIC, apart from being very transitory, all genes involved were expected for an interferon response to interferon, as shown in **Figure 11C**). In contrast, the effect of BO-110, in addition to being held, was extended to additional transcripts.

Therefore, these results illustrate intrinsic differences in the recognition and sensing of dsRNA mimics in macrophages and melanoma cells.

### Example 10. BO-110 can inhibit metastatic growth in a severely immunocompromised background.

Since melanoma cells are frequently immunoresistant, it was tested whether the direct toxicity of BO-110 towards melanoma cells was still efficient in a highly immunodeficient background. The most frequent effector mechanisms associated with melanoma immunotolerance are defects in NK, T and B cell signaling (Kirkwood et al., 2008). Therefore, the efficacy of pIC (as single agent or complexed with PEI) to block melanoma growth was tested in mice SCID Beige mice, which have impaired NK, T and B cell lymphocyte function.
To monitor treatment efficacy in the control of lung metastases, melanoma cells were labeled with GFP and injected intravenously, following the treatment schedule as described in **Fig. 10****.** B16-melanoma (**Fig. 12** panels **A, B and C**) and SK-Mel-103 (**Fig**. **12****,** panels **D** and **E**) were analyzed as representative examples of murine and human melanomas, respectively.

In both cell models, BO-110 was able to inhibit the growth of melanomas in the lung. **Fig 12A** shows the striking difference in the number of B16 metastasis visible on the lung surface after BO-110 treatment (see quantification in **Fig. 12B**). Histological analyses confirmed also the reduced number and size of B16-driven lung nodules in the BO-110 group (**Fig. 12C**). Similar analyses showed a clear anti-tumoral effect of BO-110 (but not uncomplexed pIC) in the control of disseminated growth of SK-Mel-103 (**Fig**. **12** panels **D** and **E**). In summary, our data proves a novel mode of action of a dsRNA mimic, inducing a potent anti-melanoma activity *in vivo* in SCID beige mice, which have the absence of a competent immune system (Croy and Chapeau, 1990).

### Example 11. Inhibition of metastatic growth by BO-110 human melanoma animal models.

To compare the differences between pIC and BO-110 a more relevant setting was used. Tyr::NRAS^{Q61K} x INK4a/ARF^{-/-} mice develop melanomas with similar characteristics than the human disease (Ackermann et al. 2005). Mice were treated with a single topical treatment of DMBA (7,12-dimetilbenz[a]antracene, obtained from Sigma). Once pigmented lesions reached 1 mm diameter, control PEI, naked pIC or pIC conjugated to PEI formulated for *in vivo* delivery, were administered by intraperitoneal injections (i.p) twice per week.

Again, it was observed that the antitumor activity of BO-110 was significantly higher than naked pIC as indicated by the direct measurements of tumor sizes (**Fig**. **13A** and **13B**), the metabolic activity of tumors by PET-CT (**Fig. 13C**) and the histological analysis (**Fig. 13A**).

Interesting, BO-110 doubled the time frame with no progressing lesions (**Fig. 13A**) at the treatment dosages used without secondary toxicity signals (see analysis **Fig. 13D**).

These results support the viability of treatments based in the administration of dsRNA analogues to battle the aggressive behaviors of melanoma cells.

### Example 12. Cytotoxic activity of BO-110 on a variety of tumor cells.

As the genetic and epigenetic changes present in melanoma and affecting dsRNA sensing and autophagy may not be conserved among different cancer types, it was not obvious whether BO-110 could be of therapeutic benefit in other neoplastic malignancies. In particular, tumors of pancreas, colon, bladder, brain, breast, prostate, lung and ovaries are aggressive and resistant to a variety of treatments, in part because a pleiotropic inactivation of death programs.

To define whether BO-110 could represent a novel anticancer strategy of a broad spectrum of action, a series of independently isolated cell lines pertaining to the above cited types of cancer were selected from the well-known NCI-60 panel (**Fig. 14**). Thus, altogether, these lines cover variety of tumors (i.e. pancreas, colon, bladder, breast, prostate, lung and ovarian carcinoma) of distinct genetic background. As shown in **Fig. 14****,** the analyzed cell lines had a similar sensitivity to BO-110 than the melanoma reference controls. A corollary from these data is that BO-110 is able to engage a dual induction of autophagy and apoptosis leading to a coordinated and selective killing (without affecting the viability of normal compartments) not only of melanomas, but also of cells pertaining to other different tumor types, for example: pancreas, colon, bladder, breast, prostate, lung and ovarian carcinoma.

### Example 13. BO-110 induced cell death is dependent on the activation of MDA-5, Noxa and Autophagy in tumor cell lines.

As the sensitivity to BO-110 cannot be predicted a priori (i.e. on the basis of the tumor cell type), it was necessary to define the signaling cascades mediating the response to BO-110. High throughput genetic analyses (based on cDNA arrays) in melanoma cells indicated that BO-110 was able to promote a strong upregulation of the dsRNA sensor MDA-5, as well as the proapoptotic factor NOXA. Interestingly, using immunoblotting assays, we demonstrated that in fact the sensitivity and resistance to BO-110 (e.g. in lines HCT116 or MiaPaCa2) is correlated with the ability of cells to induce MDA-5 and NOXA (**Fig. 15**). Consistent with the pro-apoptotic roles of BO-110 indicated above, sensitive cell lines showed a clear processing of caspase-9, which can be visualized as changes in electrophoretic mobility (**Fig. 15**).

### REFERENCES

1. Ackermann, J., Frutschi, M., Kaloulis, K., McKee, T., Trumpp, A., and Beermann, F. (2005). Metastasizing melanoma formation caused by expression of activated N-RasQ61K on an INK4a-deficient background. Cancer Res 65, 4005-4011
2. Akira, S., Uematsu, S., and Takeuchi, O. 2006. Pathogen recognition and innate immunity. Cell 124:783-801
3. Barral, P.M., Morrison, J.M., Drahos, J., Gupta, P., Sarkar, D., Fisher, P.B., and Racaniello, V.R. 2007. MDA-5 is cleaved in poliovirus-infected cells. J Virol 81:3677- 3684
4. Celada, A., Gray, P.W., Rinderknecht, E., and Schreiber, R.D. 1984. Evidence for a gamma-interferon receptor that regulates macrophage tumoricidal activity. J Exp Med 160:55-74
5. Chin, L., Garraway, L.A., and Fisher, D.E. 2006. Malignant melanoma: genetics and therapeutics in the genomic era. Genes Dev 20:2149-2182
6. Croy, B.A., and Chapeau, C. 1990. Evaluation of the pregnancy immunotrophism hypothesis by assessment of the reproductive performance of young adult mice of genotype scid/scid.bg/bg. J Reprod Fertil 88:231-239
7. Denoyelle, C., Abou-Rjaily, G., Bezrookove, V., Verhaegen, M., Johnson, T.M., Fullen, D.R., Pointer, J.N., Gruber, S.B., Su, L.D., Nikiforov, M.A., et al. 2006. Anti-oncogenic role of the endoplasmic reticulum differentially activated by mutations in the MAPK pathway. Nat Cell Biol 8:1053-1063
8. Fecher, L.A., Cummings, S.D., Keefe, M.J., and Alani, R.M. 2007. Toward a molecular classification of melanoma. J Clin Oncol 25:1606-1620
9. Fehrenbacher, N., and Jaattela, M. 2005. Lysosomes as targets for cancer therapy. Cancer Res 65:2993-2995
10. Fernandez, Y., Miller, T.P., Denoyelle, C., Esteban, J.A., Tang, W.H., Bengston, A.L., and Soengas, M.S. 2006. Chemical blockage of the proteasome inhibitory function of bortezomib: impact on tumor cell death. J Biol Chem 281:1107-1118
11. Fernandez, Y., Verhaegen, M., Miller, T.P., Rush, J.L., Steiner, P., Opipari, A.W., Jr., Lowe, S.W., and Soengas, M.S. 2005. Differential regulation of noxa in normal melanocytes and melanoma cells by proteasome inhibition: therapeutic implications. Cancer Res 65:6294-6304
12. Field, A.K., Tytell, A.A., Lampson, G.P., and Hilleman, M.R. 1967. Inducers of interferon and host resistance. II. Multistranded synthetic polynucleotide complexes. Proc Natl Acad Sci U S A 58:1004-1010
13. Flaherty, K.T. 2006. Chemotherapy and targeted therapy combinations in advanced melanoma. Clin Cancer Res 12:2366s-2370s
14. Gray-Schopfer, V., Wellbrock, C., and Marais, R. 2007. Melanoma biology and new targeted therapy. Nature 445:851-857
15. Hersey, P., and Zhang, X.D. 2008. Adaptation to ER stress as a driver of malignancy and resistance to therapy in human melanoma. Pigment Cell Melanoma Res 21:358-367
16. Hippert, M.M., O'Toole, P.S., and Thorburn, A. 2006. Autophagy in cancer: good, bad, or both? Cancer Res 66:9349-9351
17. Hoyer-Hansen, M., and Jaattela, M. 2008. Autophagy: an emerging target for cancer therapy. Autophagy 4:574-580
18. Ilkovitch, D., and Lopez, D.M. 2008. Immune modulation by melanoma-derived factors. Exp Dermatol.
19. Ivanov .V.N., Bhoumik A and Ronai Ze'ev. 2003. Death receptors and melanoma resistance to apoptosis. Oncogene 22:3152-3161
20. Jemal, A., Siegel, R., Ward, E., Hao, Y., Xu, J., Murray, T., and Thun, M.J. 2008. Cancer statistics, 2008. CA Cancer J Clin 58:71-96
21. Kang, D. C., Gopalkrishnan, R. V., Lin, L., Randolph, A., Valerie, K., Pestka, S., and Fisher, P. B. (2004). Expression analysis and genomic characterization of human melanoma differentiation associated gene-5, mda-5: a novel type I interferon-responsive apoptosis-inducing gene. Oncogene 23, 1789-1800
22. Kang, D.C., Gopalkrishnan, R.V., Wu, Q., Jankowsky, E., Pyle, A.M., and Fisher, P.B. 2002. mda-5: An interferon-inducible putative RNA helicase with double-stranded RNAdependent ATPase activity and melanoma growth-suppressive properties. Proc Natl Acad Sci U S A 99:637-642
23. Kawai, T., Takahashi, K., Sato, S., Coban, C., Kumar, H., Kato, H., Ishii, K.J., Takeuchi, O., and Akira, S. 2005. IPS-1, an adaptor triggering RIG-1- and Mda5-mediated type I interferon induction. Nat Immunol 6:981-988
24. Kirkwood, J.M., Tarhini, A.A., Panelli, M.C., Moschos, S.J., Zarour, H.M., Butterfield, L.H., and Gogas, H.J. 2008. Next generation of immunotherapy for melanoma. J Clin Oncol 26:3445-3455
25. Klionsky, D.J., Abeliovich, H., Agostinis, P., Agrawal, D.K., Aliev, G., Askew, D.S., Baba, M., Baehrecke, E.H., Bahr, B.A., Ballabio, A., et al. 2008. Guidelines for the use and interpretation of assays for monitoring autophagy in higher eukaryotes. Autophagy 4:151-175
26. Kovacsovics, M., Martinon, F., Micheau, O., Bodmer, J.L., Hofmann, K., and Tschopp, J. 2002. Overexpression of Helicard, a CARD-containing helicase cleaved during apoptosis, accelerates DNA degradation. Curr Biol 12:838-843
27. Kroemer, G. and Levine, B. (2008). Autophagic cell death: the story of a misnomer. Nat Rev Mol Cell Biol 9:1004-1010
28. Kroemer, G., Galluzi, L., Vandenabeele, P., Abrams, J., Alnemri, E.S., Baehrecke, E.H., Blagosklonny, M.V., El-Deity, W.S., Golstein, P., Green, D.R., et al. (2009). Classification of cell death: recommendations fo the Nomenclature Committee on Cell Death 2009. Cell Death Differ 16:3-11
29. Lev, D.C., Onn, A., Melinkova, V.O., Miller, C., Stone, V., Ruiz, M., McGary, E.C., Ananthaswamy, H.N., Price, J.E., and Bar-Eli, M. 2004. Exposure of melanoma cells to dacarbazine results in enhanced tumor growth and metastasis in vivo. J Clin Oncol 22:2092-2100
30. Lin, L., Su, Z., Lebedeva, I.V., Gupta, P., Boukerche, H., Rai, T., Barber, G.N., Dent, P., Sarkar, D., and Fisher, P.B. 2006. Activation of Ras/Raf protects cells from melanoma differentiation-associated gene-5-induced apoptosis. Cell Death Differ 13:1982-1993
31. Maiuri, M.C., Zalckvar, E., Kimchi, A., and Kroemer, G. 2007. Self-eating and selfkilling: crosstalk between autophagy and apoptosis. Nat Rev Mol Cell Biol 8:741-752
32. Mizushima, N., Levine, B., Cuervo, A.M., and Klionsky, D.J. 2008. Autophagy fights disease through cellular self-digestion. Nature 451:1069-1075
33. Payne, C.K. 2007. Imaging gene delivery with fluorescence microscopy. Nanomed 2:847-860
34. Qin, J.Z., Xin, H., Sitailo, L.A., Denning, M.F., and Nickoloff, B.J. 2006. Enhanced killing of melanoma cells by simultaneously targeting Mcl-1 and NOXA. Cancer Res 66:9636-9645
35. Qin, J.Z., Ziffra, J., Stennett, L., Bodner, B., Bonish, B.K., Chaturvedi, V., Bennett, F., Pollock, P.M., Trent, J.M., Hendrix, M.J., et al. 2005. Proteasome inhibitors trigger NOXA-mediated apoptosis in melanoma and myeloma cells. Cancer Res 65:6282-6293
36. Robinson, R.A., DeVita, V.T., Levy, H.B., Baron, S., Hubbard, S.P., and Levine, A.S. 1976. A phase I-II trial of multiple-dose polyriboinosic-polyribocytidylic acid in patieonts with leukemia or solid tumors. J Natl Cancer Inst 57:599-602
37. Schatton, T., Murphy, G.F., Frank, N.Y., Yamaura, K., Waaga-Gasser, A.M., Gasser, M., Zhan, Q., Jordan, S., Duncan, L.M., Weishaupt, C., et al. 2008. Identification of cells initiating human melanomas. Nature 451:345-349
38. Soengas, M.S., and Lowe, S.W. 2003. Apoptosis and melanoma chemoresistance. Oncogene 22:3138-3151
39. Soengas, M.S., Capodieci, P., Polsky, D., Mora, J., Esteller, M., Opitz-Araya, X., McCombie, R., Herman, J.G., Gerald, W.L., Lazebnik, Y.A., et al. 2001. Inactivation of the apoptosis effector Apaf-1 in malignant melanoma. Nature 409:207-211
40. Soengas, M.S., Gerald, W.L., Cordon-Cardo, C., Lazebnik, Y., and Lowe, S.W. 2006. Apaf-1 expression in malignant melanoma. Cell Death Differ 13:352-353
41. Tawbi, H.A., and Kirkwood, J.M. 2007. Management of metastatic melanoma. Semin Oncol 34:532-545
42. Tormo, D., Ferrer, A., Bosch, P., Gaffal, E., Basner-Tschakarjan, E., Wenzel, J., and Tuting, T. 2006. Therapeutic efficacy of antigen-specific vaccination and toll-like receptor stimulation against established transplanted and autochthonous melanoma in mice. Cancer Res 66:5427-5435
43. Verma, S., Petrella, T., Hamm, C., Bak, K., and Charette, M. 2008. Biochemotherapy for the treatment of metastatic malignant melanoma: a clinical practice guideline. Curr Oncol 15:85-89
44. Wenzel, J., Tormo, D., and Tuting, T. 2008. Toll-like receptor-agonists in the treatment of skin cancer: history, current developments and future prospects. Handb Exp Pharmacol:201-220
45. Wilcox, R., and Markovic, S.N. 2007. Tumor immunotherapy in melanoma: on the dawn of a new era? Curr Opin Mol Ther 9:70-78
46. Wolter, K.G., Verhaegen, M., Fernandez, Y., Nikolovska-Coleska, Z., Riblett, M., de la Vega, C.M., Wang, S., and Soengas, M.S. 2007. Therapeutic window for melanoma treatment provided by selective effects of the proteasome on Bcl-2 proteins. Cell Death Differ 14:1605-1616
47. Xie, Z., and Klionsky, D.J. 2007. Autophagosome formation: core machinery and adaptations. Nat Cell Biol 9:1102-1109
48. Yoneyama, M., Kikuchi, M., Matsumoto, K., Imaizumi, T., Miyagishi, M., Taira, K., Foy, E., Loo, Y.M., Gale, M., Jr., Akira, S., et al. 2005. Shared and unique functions of the DExD/H-box helicases RIG-I, MDA5, and LGP2 in antiviral innate immunity. J Immunol 175:2851-2858

## Claims

1. A process for the identification of compounds to be used as therapeutic agents for treating cancer comprising the steps of:
a) Contacting the candidate compound with a cancer cell culture, or cancer cell line derived from cancer cells;
b) Determining the level of activation of a family helicase MDA-5 or the level of NOXA expression, in combination with the determination of the induction of autophagy in cancer cells or in a cell line derived from cancer cells;
c) Comparing the data obtained in step b) with those observed in control of the same cells treated similarly, but in the absence of the candidate compound;
d) Selecting the compounds which have given rise to a significant increase in the parameter or parameters determined in step b) in comparison with the control.

2. The process, according to claim 1, in which the determination of the autophagy induction is performed by checking the level of expression, the presence of posttranslational modifications or intracellular localization of a protein autophagy.

3. The process, according to claim 1, in which the induction of autophagy is determined by a technique selected from the group:
i. Change of electrophoretic mobility of the protein LC3, or
ii. Detection of foci formation of protein LC3.

4. The process, according to claim 1, in which the induction of autophagy is determined by checking the presence of autophagosomes by microscopic observation thereof.

5. The process, according to claim 4, in which the presence of autophagosomes is checked using transmission electron microscopy.

6. The process, according to claim 1, in which the activation level of MDA-5, the level of expression of NOXA and induction of autophagy are determined.

7. The process, according to claim 1, for the identification of compounds to be used as therapeutic agents for treating melanoma comprising the steps of:
a) Contacting the candidate compound with a melanoma cell culture, or a cell line derived from melanoma cells;
b) Determining the level of activation of a family helicase MDA-5 or the level of NOXA expression, in combination with the determination of the induction of autophagy in cancer cells or in a cell line derived from cancer cells;
c) Comparing the data obtained in step b) with those observed in control of the same cells treated similarly, but in the absence of the candidate compound;
d) Selecting the compounds which have given rise to a significant increase in the parameter or parameters determined in step b) in comparison with the control.

8. The process, according to claim 7, wherein the cell line is selected from the group of human cell lines: SK-Mel-19, SK-Mel-28. SK-Mel-103 and SK-Mel-147, and B16 mouse cells.

9. The process, according to claim1, for the identification of compounds to be used as therapeutic agents for treating at least one of the following types of cancer: pancreas, colon, bladder, breast, prostate, lung and ovarian carcinoma comprising the steps of:
a) Contacting the candidate compound with a cancer cell culture from at least one of the above cited types of cancer, or a cell line derived from at least one of the above cited types of cancer;
b) Determining the level of activation of a family helicase MDA-5 or the level of NOXA expression, in combination with the determination of the induction of autophagy in cancer cells or in a cell line derived from cancer cells;
c) Comparing the data obtained in step b) with those observed in control of the same cells treated similarly, but in the absence of the candidate compound;
d) Selecting the compounds who have given rise to a significant increase in the parameter or parameters determined in step b) in comparison with the control.

10. The process, according to claim 9, wherein the cell line is selected from the group of pancreas cancer cell lines: IMIMPC2, MiaPaCa2, Aspc1, A6L, SKPC1 and Panc-1; or from the group of colon cancer cell lines: CACO, SW480 and SW1222; or from the group of bladder cancer cell lines: RT112, MGHU4, 639V, 253J, MGHu3 and SW1170; or from the group of glioma cell lines: U87MG, U251 and T98G; or from the group of breast cancer cell lines: MDA231, MCF7 and T47D; or from the group of prostate cancer cell lines: LNCaP, PC3 and DU145; or from the group of lung cancer cell lines: H1299 and NCI H460; or from the group of ovarian cancer cells lines: NCI H23, CHQK1 and SK-OV-3.

11. The process, according to claims 1 to 10, wherein the selected compound consists of a combination of double-stranded RNA (dsRNA) which is at least 1000 nucleotides per chain in length and a polycation.

12. The process, according to claim 11, wherein the selected compound consists of a combination of polyinosine-polycytidylic acid (pIC) which is at least 1000 nucleotides per chain in length and polyethyleneimine (PEI).

13. Compound consisting of a combination of polyinosine-polycytidylic acid (pIC) which is at least 1000 nucleotides per chain in length and polyethyleneimine (PEI) for use as medicament.

14. Compound, according to claim 13, consisting of a combination of polyinosine-polycytidylic acid (pIC) which is at least 1000 nucleotides per chain in length and polyethyleneimine (PEI) for use in the treatment of cancer.

15. Pharmaceutical composition comprising the compound of the claims 13 or 14 for use as medicament.

16. Pharmaceutical composition comprising the compound of the claims 13 or 14, according to claim 15, for use in the treatment of cancer.

17. Pharmaceutical composition of claim 16 for use in the treatment of a type of cancer **characterized by** showing the activation of the biomarker MDA-5 helicase or the expression of NOXA, in combination with biomarkers related with the induction of autophagy.

18. Pharmaceutical composition, according to claim 16, for use in the treatment of melanoma.

19. Pharmaceutical composition, according to claim 16, for use in the treatment of at least one of the following types of cancer: pancreas, colon, bladder, breast, prostate, lung and ovarian carcinoma.

## Patentansprüche

1. Ein Verfahren zum Identifizieren von Verbindungen, die als therapeutische Wirkstoffe zur Behandlung von Krebs verwendet werden, bestehend aus den folgenden Schritten:
a) Inkontaktbringen der Kandidatenverbindung mit einer Krebszellkultur oder mit einer Krebszelllinie, die von Krebszellen abgeleitet wurde;
b) Bestimmen des Aktivierungsgrades der Familie Helikase MDA-5 oder des Grades der NOXA-Expression, in Verbindung mit dem Bestimmen der Induktion einer Autophagie in Krebszellen oder in einer Zelllinie, die von Krebszellen abgeleitet wurde;
c) Vergleichen der bei Schritt b) gewonnenen Daten mit denen, die man bei der Kontrolle der gleichen Zellen, die vergleichbar, jedoch in Abwesenheit der Kandidatenverbindung behandelt wurden, erhalten hat;
d) Auswählen der Verbindungen, die im Vergleich zur Kontrolle zu einem signifikanten Anstieg des Parameters bzw. der Parameter, die bei Schritt b) bestimmt wurden, geführt haben.

2. Das Verfahren nach Anspruch 1, bei dem das Bestimmen der Autophagieinduktion durchgeführt wird, indem der Expressionsgrad, das Vorhandensein von posttranslationalen Modifikationen oder die intrazelluläre Lokalisierung einer Proteinautophagie überprüft wird.

3. Das Verfahren nach Anspruch 1, bei dem die Induktion der Autophagie mit einer Technik bestimmt wird, die aus der folgenden Gruppe ausgewählt ist:
i. Verändern der elektrophoretischen Mobilität des Proteins LC3, oder
ii. Feststellen einer Focibildung des Proteins LC3.

4. Das Verfahren nach Anspruch 1, bei dem die Induktion der Autophagie bestimmt wird, indem das Vorhandensein von Autophagosomen durch mikroskopisches Beobachten überprüft wird.

5. Das Verfahren nach Anspruch 4, bei dem das Vorhandensein von Autophagosomen unter Verwendung der Transmissionselektronenmikroskopie überprüft wird.

6. Das Verfahren nach Anspruch 1, bei dem der Aktivierungsgrad von MDA-5, der Expressionsgrad von NOXA und die Induktion der Autophagie bestimmt werden.

7. Ein Verfahren nach Anspruch 1, zum Identifizieren von Verbindungen, die als therapeutische Wirkstoffe zur Behandlung von Melanom verwendet werden, bestehend aus den folgenden Schritten:
a) Inkontaktbringen der Kandidatenverbindung mit einer Melanomzellkultur oder mit einer Zelllinie, die von Melanomzellen abgeleitet wurde;
b) Bestimmen des Aktivierungsgrades der Familie Helikase MDA-5 oder des Grades der NOXA-Expression, in Verbindung mit dem Bestimmen der Induktion einer Autophagie in Krebszellen oder in einer Zelllinie, die von Krebszellen abgeleitet wurde;
c) Vergleichen der bei Schritt b) gewonnenen Daten mit denen, die man bei der Kontrolle der gleichen Zellen, die vergleichbar, jedoch in Abwesenheit der Kandidatenverbindung behandelt wurden, erhalten hat;
d) Auswählen der Verbindungen, die im Vergleich zur Kontrolle zu einem signifikanten Anstieg des Parameters bzw. der Parameter, die bei Schritt b) bestimmt wurden, geführt haben.

8. Das Verfahren nach Anspruch 7, bei dem die Zelllinie aus der folgenden Gruppe menschlicher Zelllinien ausgewählt ist: SK-Mel-19, SK-Mel-28. SK-Mel-103 und SK-Mel-147, und B16-Mauszellen.

9. Das Verfahren nach Anspruch 1, zum Identifizieren von Verbindungen, die als therapeutische Wirkstoffe zur Behandlung von mindestens einer der folgenden Krebsarten verwendet werden: Pankreas-, Kolon-, Blasen-, Mamma-, Prostata-, Lungen- und Ovarialkarzinom, bestehend aus den folgenden Schritten:
a) Inkontaktbringen der Kandidatenverbindung mit einer Krebszellkultur von mindestens einer der oben genannten Krebsarten oder mit einer Zelllinie, die von einer der oben genannten Krebsarten abgeleitet wurde;
b) Bestimmen des Aktivierungsgrades der Familie Helikase MDA-5 oder des Grades der NOXA-Expression, in Verbindung mit dem Bestimmen der Induktion einer Autophagie in Krebszellen oder in einer Zelllinie, die von Krebszellen abgeleitet wurde;
c) Vergleichen der bei Schritt b) gewonnenen Daten mit denen, die man bei der Kontrolle der gleichen Zellen, die vergleichbar, jedoch in Abwesenheit der Kandidatenverbindung behandelt wurden, erhalten hat;
d) Auswählen der Verbindungen, die im Vergleich zur Kontrolle zu einem signifikanten Anstieg des Parameters bzw. der Parameter, die bei Schritt b) bestimmt wurden, geführt haben.

10. Das Verfahren nach Anspruch 9, bei dem die Zelllinie aus der folgenden Gruppe von Pankreastumorzelllinien ausgewählt ist: IMIMPC2, MiaPaCa2, Aspcl, A6L, SKPC1 und Panc-1; oder aus der folgenden Gruppe von Kolonkrebszelllinien: CACO, SW480 und SW1222; oder aus der folgenden Gruppe von Blasenkrebszelllinien: RT112, MGHU4,639V, 253J, MGHu3 und SW1170; oder aus der folgenden Gruppe von Gliomzelllinien: U87MG, U251 und T98G; oder aus der folgenden Gruppe von Brustkrebszelllinien: MDA231, MCF7 und T47D; oder aus der folgenden Gruppe von Prostatakrebszelllinien: LNCaP, PC3 und DU145; oder aus der folgenden Gruppe von Lungenkrebszelllinien: H1299 und NCI H460; oder aus der folgenden Gruppe von Eierstockkrebszelllinien: NCI H23, CHQK1 und SK-OV-3.

11. Das Verfahren nach den Ansprüchen 1 bis 10, wobei die ausgewählte Verbindung aus einer Kombination einer doppelsträngigen RNA (dsRNA), die pro Kette mindestens 1000 Nukleotiden lang ist, und einem Polykation besteht.

12. Das Verfahren nach Anspruch 11, wobei die ausgewählte Verbindung aus einer Kombination einer polyinosinischen-polycytidylischen Säure (plC), die pro Kette mindestens 1000 Nukleotiden lang ist, und einem Polyethylenimin (PEI) besteht.

13. Verbindung, bestehend aus einer Kombination einer polyinosinischenpolycytidylischen Säure (plC), die pro Kette mindestens 1000 Nukleotiden lang ist, und einem Polyethylenimin (PEI), zur Verwendung als Medikament.

14. Verbindung nach Anspruch 13, bestehend aus einer Kombination einer polyinosinischen-polycytidylischen Säure (plC), die pro Kette mindestens 1000 Nukleotiden lang ist, und einem Polyethylenimin (PEI), zur Verwendung bei der Behandlung von Krebs.

15. Pharmazeutische Zusammensetzung, bestehend aus der Verbindung der Ansprüche 13 oder 14, zur Verwendung als Medikament.

16. Pharmazeutische Zusammensetzung, bestehend aus der Verbindung der Ansprüche 13 oder 14, nach Anspruch 15, zur Verwendung bei der Behandlung von Krebs.

17. Pharmazeutische Zusammensetzung nach Anspruch 16, zur Verwendung bei der Behandlung einer Krebsart, die **dadurch gekennzeichnet ist, dass** sie die Aktivierung des Biomarkers MDA-5 Helikase oder die Expression von NOXA aufweist, in Verbindung mit Biomarkern, die im Zusammenhang mit der Induktion von Autophagie stehen.

18. Pharmazeutische Zusammensetzung nach Anspruch 16, zur Verwendung bei der Behandlung von Melanom.

19. Pharmazeutische Zusammensetzung nach Anspruch 16, zur Verwendung bei der Behandlung von mindestens einer der folgenden Krebsarten: Pankreas-, Kolon-, Blasen-, Mamma-, Prostata-, Lungen- und Ovarialkarzinom.

## Revendications

1. Un procédé pour l'identification de composés devant être utilisés comme agents thérapeutiques pour le traitement du cancer comprenant les étapes de :
a) Mise en contact du composé candidat avec une culture de cellules cancéreuses, ou une lignée cellulaire cancéreuse dérivée de cellules cancéreuses ;
b) Détermination du niveau d'activation d'une hélicase de famille MDA-5 ou du niveau d'expression de NOXA, en association avec la détermination de l'induction d'autophagie dans des cellules cancéreuses ou dans une lignée cellulaire dérivée de cellules cancéreuses ;
c) Comparaison des données obtenues dans l'étape b) avec celles observées dans le témoin des mêmes cellules traitées de façon similaire, mais en l'absence du composé candidat ;
d) Sélection des composés qui ont donné lieu à une augmentation significative dans le paramètre ou les paramètres déterminés dans l'étape b) par comparaison avec le témoin.

2. Le procédé, selon la revendication 1, dans lequel la détermination de l'induction d'autophagie est réalisée par la vérification du niveau d'expression, de la présence de modifications post-transrationnelles ou la localisation intracellulaire d'une autophagie de protéine.

3. Le procédé, selon la revendication 1, dans lequel l'induction d'autophagie est déterminée par une technique sélectionnée dans le groupe :
i. Changement de mobilité électrophorétique de la protéine LC3, ou
ii. Détection de la formation de foyers de protéine LC3.

4. Le procédé, selon la revendication 1, dans lequel l'induction d'autophagie est déterminée par la vérification de la présence d'autophagosomes par leur observation au microscope.

5. Le procédé, selon la revendication 4, dans lequel la présence d'autophagosomes est vérifiée en utilisant la microscopie électronique par transmission.

6. Le procédé, selon la revendication 1, dans lequel le niveau d'activation de MDA-5, le niveau d'expression de NOXA et l'induction d'autophagie sont déterminés.

7. Le procédé, selon la revendication 1, pour l'identification de composés devant être utilisés comme agents thérapeutiques pour le traitement de mélanome comprenant les étapes de :
a) Mise en contact du composé candidat avec une culture de cellules de mélanome, ou une lignée cellulaire dérivée de cellules de mélanome ;
b) Détermination du niveau d'activation d'une hélicase de famille MDA-5 ou du niveau d'expression de NOXA, en association avec la détermination de l'induction d'autophagie dans des cellules cancéreuses ou dans une lignée cellulaire dérivée de cellules cancéreuses ;
c) Comparaison des données obtenues dans l'étape b) avec celles observées dans le témoin des mêmes cellules traitées de façon similaire, mais en l'absence du composé candidat ;
d) Sélection des composés ayant donné lieu à une augmentation significative dans le paramètre ou les paramètres déterminés dans l'étape b) par comparaison avec le témoin.

8. Le procédé, selon la revendication 7, dans lequel la lignée cellulaire est sélectionnée dans le groupe de lignées cellulaires humaines : SK-Mel-19, SK-Mel-28. SK-Mel-103 et SK-Mel-147, et des cellules de souris B16.

9. Le procédé, selon la revendication 1, pour l'identification de composés devant être utilisés comme agents thérapeutiques pour le traitement d'au moins l'un des types de cancer suivants : pancréas, côlon, vessie, sein, prostate, poumon et carcinome de l'ovaire comprenant les étapes de :
a) Mise en contact du composé candidat avec une culture de cellules cancéreuses d'au moins l'un des types de cancer mentionnés ci-dessus, ou une lignée cellulaire dérivée d'au moins l'un des types de cancer mentionnés ci-dessus ;
b) Détermination du niveau d'activation d'une hélicase de famille MDA-5 ou du niveau d'expression de NOXA, en association avec la détermination de l'induction d'autophagie dans des cellules cancéreuses ou dans une lignée cellulaire dérivée de cellules cancéreuses ;
c) Comparaison des données obtenues dans l'étape b) avec celles observées dans le témoin des mêmes cellules traitées de façon similaire, mais en l'absence du composé candidat ;
d) Sélection des composés qui ont donné lieu à une augmentation significative dans le paramètre ou les paramètres déterminés dans l'étape b) par comparaison avec le témoin.

10. Le procédé, selon la revendication 9, dans lequel la lignée cellulaire est sélectionnée dans le groupe des lignées cellulaires de cancer du pancréas : IMIMPC2, MiaPaCa2, Aspcl, A6L, SKPC1 et Panc-1 ; ou dans le groupe des lignées cellulaires de cancer du côlon : CACO, SW480 et SW1222 ; ou dans le groupe des lignées cellulaires de cancer de la vessie : RT112, MGHU4,639V, 253J, MGHu3 et SW1170 ; ou dans le groupe des lignées cellulaires de gliome : U87MG, U25X et T98G ; ou dans le groupe des lignées cellulaires de cancer du sein : MDA231, MCF7 et T47D ; ou dans le groupe des lignées cellulaires de cancer de la prostate : LNCaP, PC3 et DU145 ; ou dans le groupe des lignées cellulaires de cancer du poumon : HI299 et NCI H460 ; ou dans le groupe des lignées cellulaires de cancer de l'ovaire : NCI H23, CHQK1 et SK-OV-3.

11. Le procédé, selon les revendications 1 à 10, dans lequel le composé sélectionné consiste en une combinaison d'ARN double brin (ARNdb) qui est au moins de 1000 nucléotides par chaîne en longueur et une polycation.

12. Le procédé, selon la revendication 11, dans lequel le composé sélectionné consiste en une combinaison de polyinosine-acide polycytidylique (pIC) qui est au moins de 1000 nucléotides par chaîne en longueur et de polyéthylèneimine (PEI).

13. Composé consistant en une combinaison de polyinosine-acide polycytidylique (pIC) qui est au moins de 1000 nucléotides par chaîne en longueur et de polyéthylèneimine (PEI) à utiliser comme médicament.

14. Composé, selon la revendication 13, consistant en une combinaison de polyinosine- acide polycytidylique (pIC) qui est au moins de 1000 nucléotides par chaîne en longueur et de polyéthylèneimine (PEI) à utiliser pour le traitement du cancer.

15. Composition pharmaceutique comprenant le composé des revendications 13 ou 14 à utiliser comme médicament.

16. Composition pharmaceutique comprenant le composé des revendications 13 ou 14, selon la revendication 15, à utiliser dans le traitement du cancer.

17. Composition pharmaceutique de la revendication 16 à utiliser dans le traitement d'un type de cancer, **caractérisée par le fait qu'**elle montre l'activation du biomarqueur hélicase MDA-5 ou l'expression de NOXA, en association avec des biomarqueurs liés à l'induction d'autophagie.

18. Composition pharmaceutique, selon la revendication 16, à utiliser dans le traitement du mélanome ;

19. Composition pharmaceutique, selon la revendication 16, à utiliser dans le traitement d'au moins l'un des types de cancer suivants : pancréas, côlon, vessie, sein, prostate, poumon et carcinome de l'ovaire.
